(19) Europäisches Patentamt
European Patent Office
Office européen des brevets

(11) Veröffentlichungsnummer: **0 235 558 A2**

(12) **EUROPÄISCHE PATENTANMELDUNG**

(21) Anmeldenummer: **87101037.7**

(22) Anmeldetag: **26.01.87**

(51) Int. Cl.⁴: **C07D 231/40**, C07D 231/38, A01N 43/56

(30) Priorität: **04.02.86 DE 3603291**

(43) Veröffentlichungstag der Anmeldung: **09.09.87 Patentblatt 87/37**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Gehring, Reinhold, Dr.**
**Dasnöckel 49**
**D-5600 Wuppertal 11(DE)**
Erfinder: **Schallner, Otto, Dr.**
**Noldeweg 22**
**D-4018 Monheim(DE)**
Erfinder: **Stetter, Jörg, Dr.**
**Gellertweg 4**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Lürssen, Klaus, Dr.**
**August-Kierspel-Strasse 151**
**D-5060 Bergisch Gladbach 2(DE)**

(54) **5-Amino-1-phenyl-pyrazole.**

(57) Die Erfindung betrifft 5-Amino-1-phenyl-pyrazole der Formel I

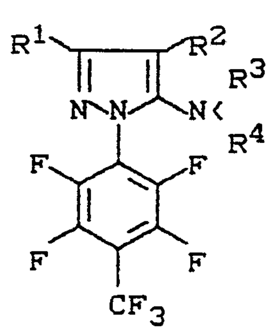

(I)

in welcher
$R^1$ für Wasserstoff oder für Alkyl mit 1 bis 12 Kohlenstoffatomen steht,
$R^2$ für Wasserstoff, Nitro, Nitroso, Halogen oder für den Rest $-\underset{\underset{O}{\|}}{C}-R^5$

EP 0 235 558 A2

steht, wobei

$R^3$ für Wasserstoff, für einen Rest

$-\overset{X}{\overset{\|}{C}}-R^6$ oder für einen Rest $-S(O)_n-R^7$ steht,

$R^4$ für Wasserstoff, für Alkyl, für einen Rest

$-\overset{X}{\overset{\|}{C}}-R^6$ oder für einen Rest $-S(O)_n-R^7$ steht und für den Fall, daß $R^3$ für einen $-SO_2-R^7$-Rest oder einen $-CO-C_mF_{2m+1}$Rest steht auch für ein salzartig gebundenes anorganisches oder organisches Kation steht,

wobei jedoch für den Fall, daß $R^1$ und $R^3$ für Wasserstoff stehen und $R^2$ für Nitro steht, $R^4$ nicht gleichzeitig für einen Propionylrest steht, und wobei $R^5$, $R^6$, $R^7$, X, n und m die in der Beschreibung angegebene Definitionen haben,

mehrere Verfahren zu ihrer Herstellung und ihre Verwendung als Herbizide und Wachstumsregulatoren.

# 5-Amino-1-phenyl-pyrazole

Die Erfindung betrifft neue 5-Amino-1-phenyl-pyrazole, mehrere Verfahren zu ihrer Herstellung und ihre Verwendung als Heribizide und Pflanzenwachstumsregulatoren.

Es ist bekannt, daß bestimmte 5-Amino-1-phenyl-pyrazole, wie beispielsweise das 4-Nitro-5-propionamido-1-(2,3,5,6-tetrafluor-4-trifluormethyl-phenyl)-pyrazol herbizide, insbesondere auch selektiv-herbizide Eigenschaften besitzen (vergl. z.B. DE-OS 34 02 308).

Deren herbizide Wirkung gegenüber Schadpflanzen ist jedoch ebenso wie ihre Verträglichkeit gegenüber wichtigen Kulturpflanzen nicht immer in allen Anwendungsbereichen völlig zufriedenstellend.

Es wurden neue 5-Amino-1-phenyl-pyrazole der allgemeinen Formel (I),

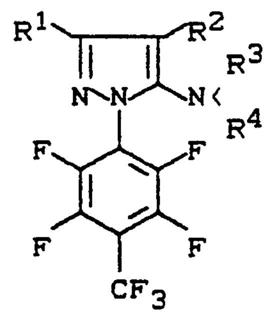

(I)

in welcher

$R^1$ für Wasserstoff oder für Alkyl mit 1 bis 12 Kohlenstoffatomen steht,

$R^2$ für Wasserstoff, Nitro, Nitroso, Halogen oder für einen Rest $-\underset{\underset{O}{\|}}{C}-R^5$ steht, wobei

$R^5$ für Wasserstoff, Hydroxy, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

$R^3$ für Waserstoff, für einen Rest $-\overset{\overset{X}{\|}}{C}-R^6$ oder für einen Rest $-S(O)_n-R^7$, steht,

$R^4$ für Wasserstoff, für Alkyl, für einen Rest $-\overset{\overset{X}{\|}}{C}-R^6$ oder für einen Rest $-S(O)_n-R^7$ steht, und für den Fall, daß $R^3$ für einen $-SO_2-R^7$-Rest oder einen $-CO-C_m F_{2m+1}$-Rest steht, auch für ein salzartig gebundenes anorganisches oder organisches Kation steht, wobei

$R^6$ für Wasserstoff, Alkyl, Alkenyl, Alkinyl, Halogenalkyl, Alkoxyalkyl, Alkylthioalkyl, Alkylsulfonylalkyl, Alkylsulfinylalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

X für Sauerstoff oder Schwefel steht,

n für eine Zahl 0, 1 oder 2 steht,

m für eine Zahl 1, 2 oder 3 steht und

$R^7$ für Alkyl, Halogenalkyl oder für gegebenenfalls substituiertes Aryl steht,

wobei jedoch für den Fall, daß $R^1$ und $R^3$ für Wasserstoff stehen und $R^2$ für Nitro steht, $R^4$ nicht gleichzeitig für einen Propionylrest steht,

gefunden.

Weiterhin wurde gefunden, daß sich die neuen 5-Amino-1-phenyl-pyrazole der allgemeinen Formel (I),

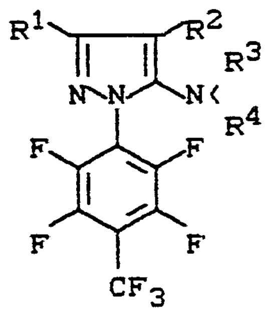

(I)

in welcher
$R^1$ für Wasserstoff oder für Alkyl mit 1 bis 12 Kohlenstoffatomen steht,
$R^2$ für Wasserstoff, Nitro, Nitroso, Halogen oder für den Rest $-\underset{\underset{O}{\|}}{C}-R^5$ steht, wobei
$R^5$ für Wasserstoff, Hydroxy, Alkyl, Alkenyl, Alkinyl, Halogenalkyl, Alkoxyalkyl Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,
$R^3$ für Wasserstoff, für einen Rest $-\overset{\overset{X}{\|}}{C}-R^6$ oder für einen Rest $-S(O)_n-R^7$ steht,
$R^4$ für Wasserstoff, für Alkyl, für einen Rest $-\overset{\overset{X}{\|}}{C}-R^6$ oder für einen Rest $-S(O)_n-R^7$ steht und für den Fall, daß $R^3$ für einen $-SO_2-R^7$-Rest oder einen $-CO-C_mF_{2m+1}$ Rest steht auch für ein salzartig gebundenes anorganisches oder organisches Kation steht,
$R^6$ für Wasserstoff, Alkyl, Alkenyl, Alkinyl, Halogenalkyl, Alkoxyalkyl, Alkylthioalkyl, Alkylsulfonylalkyl, Alkylsulfinylalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,
X für Sauerstoff oder Schwefel steht,
n für eine Zahl 0, 1 oder 2 steht,
m für eine Zahl 1, 2 oder 3 steht und
$R^7$ für Alkyl, Halogenalkyl oder für gegebenenfalls substituiertes Aryl steht,
wobei jedoch für den Fall, daß $R^1$ und $R^3$ für Wasserstoff stehen und $R^2$ für Nitro steht, $R^4$ nicht gleichzeitig für einen Propionylrest steht,
nach folgenden Verfahren herstellen lassen:

Man erhält

a) die erfindungsgemäßen 5-Amino-1-phenyl-pyrazol-Derivate der Formel (Ia)

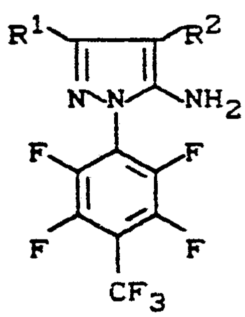

(Ia)

in welcher
$R^1$ und $R^2$ die oben angegebene Bedeutung haben,
wenn man 2,3,5,6-Tetrafluor-4-trifluormethyl-phenylhydrazin der Formel (II),

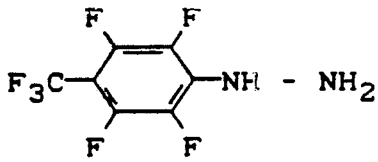

(II)

mit Acrylnitril-Derivaten der Formel (III),

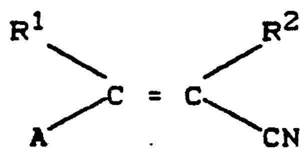

(III)

in welcher
R¹ und R² die oben angegebene Bedeutung haben und
A für Halogen, Hydroxy, Alkoxy oder Dialkylamino steht,
zunächst in einer 1. Stufe gegebenenfalls in Gegenwart eines Verdünnungsmittels und gegebenenfalls in Gegenwart eines Reaktionshilfsmittels umsetzt zu den Phenylhydrazin-Derivaten der Formel (IV),

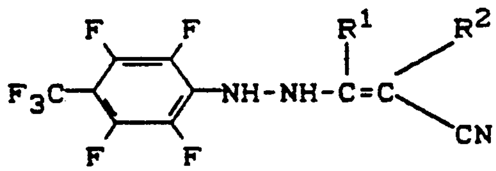

(IV)

in welcher
R¹ und R² die oben angegebene Bedeutung haben,
und dieser in einer 2. Stufe, gegebenenfalls in Gegenwart eines Verdünnungsmittels und gegebenenfalls in Gegenwart eines Säurebindemittels cyclisiert,
oder man erhält

b) die erfindungsgemäßen 5-Amino-1-phenyl-pyrazol-Derivate der Formel (Ib),

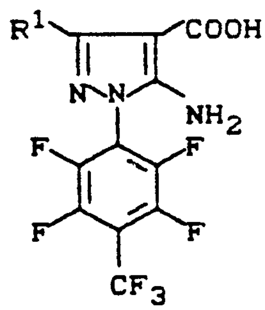

(Ib)

in welcher
R¹ die oben angegebene Bedeutung hat,
wenn man 4-Alkoxycarbonyl-5-amino-pyrazole der Formel (Ir)

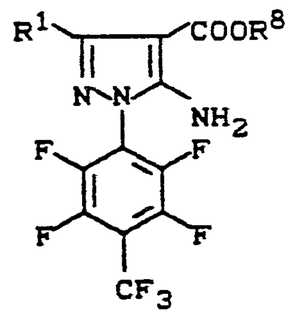

(Ir)

in welcher
$R^1$ die oben angegebene Bedeutung hat und
$R^8$ für Alkyl steht,
an der Estergruppe in 4-Position des Pyrazolringes in allgemein üblicher Art und Weise, gegebenenfalls in Gegenwart eines Verdünnungsmittels und gegebenenfalls in Gegenwart eines Katalysators, verseift,
oder man erhält

c) die erfindungsgemäßen 5-Amino-1-phenyl-pyrazol-Derivate der Formel (Ic),

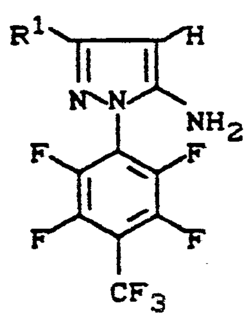

(Ic)

in welcher
$R^1$ die oben angegebene Bedeutung hat,
wenn man 5-Amino-1-phenyl-pyrazol-Derivate der Formel (Ib),

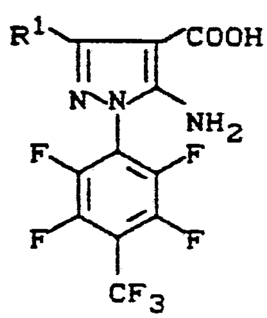

(Ib)

in welcher
$R^1$ die oben angegebene Bedeutung hat,
in allgemein üblicher Weise, gegebenenfalls in Gegenwart eines Verdünnungsmittels und gegebenenfalls in Gegenwart eines Katalysators decarboxyliert,
oder man erhält

d) die erfindungsgemäßen 5-Amino-1-phenyl-pyrazol-Derivate der Formel (Id),

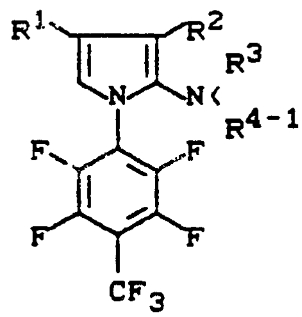

(Id)

in welcher
$R^{4-1}$ für Alkyl, für einen Rest

X
‖
- C -$R^6$ oder für einen Rest -S(O) $_n$-$R^7$ steht und

$R^1$, $R^2$, $R^3$ , $R^6$, $R^7$, X und n die oben angegebene Bedeutung haben,
wenn man 5-Amino-1-phenyl-pyrazole der Formel (Is)

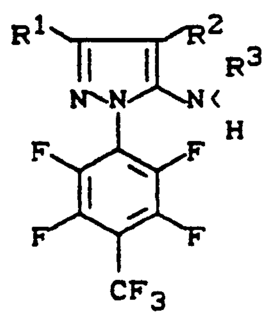

(Is)

in welcher
$R^1$, $R^2$ und $R^3$ die oben angegebene Bedeutung haben,
(d-α) mit Verbindungen der Formel (V),

X
‖
$R^6$- C -$A^1$ (V)

in welcher
$A^1$ für Halogen oder für einen Rest

O
‖
$R^6$- C -O-steht und

$R^6$ und X die oben angegebene Bedeutung haben,
oder
(d-β) mit Verbindungen der Formel (Va),
$R^7$-S(O)$_n$-$A^2$ (Va)
in welcher
$A^2$ für Halogen steht und
$R^7$ und n die oben angegebene Bedeutung haben,
oder
(d-γ) mit Verbindungen der Formel (Vb),
$R^{8-1}$-$A^3$ (Vb)
in welcher
$R^{8-1}$ für Alkyl steht und
$A^3$ für Halogen, p-Toluolsulfonyloxy oder Alkoxysulfonyloxy steht,
gegebenenfalls in Gegenwart eines Verdünnungsmittels und gegebenenfalls in Gegenwart eines Säurebindemittels umsetzt,
oder man erhält

e) die erfindungsgemäßen 5-Amino-1-phenyl-pyrazol-Derivate der Formel (Ie),

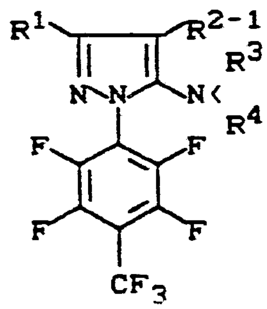

(Ie)

in welcher
$R^{2-1}$ für Halogen, Nitro, Nitroso, Formyl, Alkanoyl oder Aroyl steht und
$R^1$, $R^3$ und $R^4$ die oben angegebene Bedeutung haben,
wenn man 5-Amino-1-phenyl-pyrazol-Derivate der Formel (It),

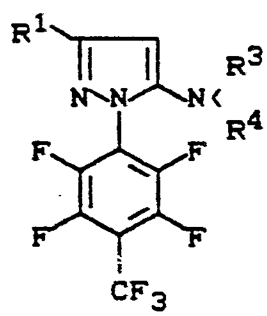

(It)

in welcher
$R^1$, $R^3$ und $R^4$ die oben angegebene Bedeutung haben,
mit elektrophilen Agenzien der Formel (VI),
$R^{2-1}$-$A^4$ (VI)
in welcher
$A^4$ für eine elektronenanziehende Abgangsgruppe steht und
$R^{2-1}$ die oben angegebene Bedeutung hat,
oder mit anderen üblichen elektrophilen Agenzien gegebenenfalls in Gegenwart eines Verdünnungsmittels und gegebenenfalls in Gegenwart eines Katalysators oder Reaktionshilfsmittels in 4-Stellung substituiert, oder man erhält

f) die erfindungsgemäßen 5-Amino-1-phenyl-pyrazol-Derivate der Formel (If)

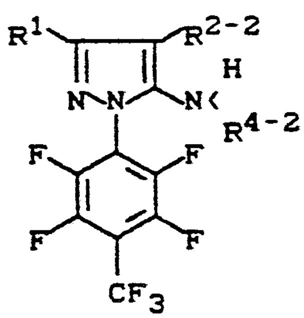

(If)

in welcher
$R^{2-2}$ für Halogen, Nitro oder Nitroso steht,
$R^{4-2}$ für Wasserstoff oder Alkyl steht und
$R^1$ die oben angegebene Bedeutung hat,
wenn man 5-Acylamino-1-phenyl-pyrazole der Formel (Iu),

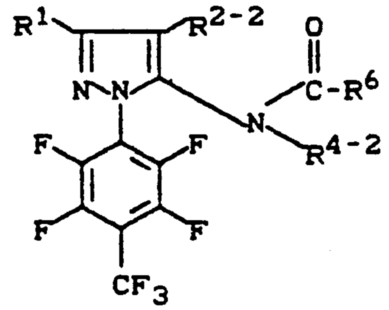

(Iu)

in welcher
R', $R^{2-2}$, $R^{4-2}$ und $R^6$ die oben angegebene Bedeutung haben,
gegebenenfalls in Gegenwart eines Verdünnungsmittels und gegebenenfalls in Gegenwart eines Katalysators an der Aminogruppe in 5-Position des Pyrazolringes deacyliert,
oder man erhält

g) die erfindungsgemäßen 5-Amino-1-phenyl-pyrazol-Derivate der Formel (Ig),

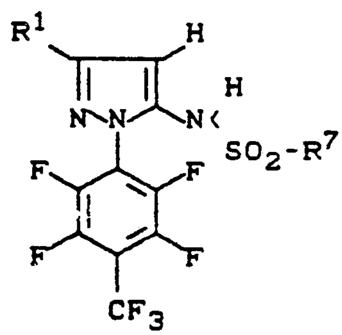

(Ig)

in welcher
R' und $R^7$ die oben angegebene Bedeutung haben,
wenn man 5-Bis-sulfonyl-amino-pyrazole der Formel (Iv),

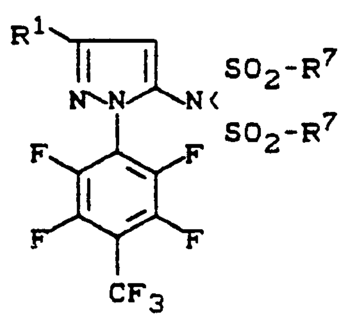

(Iv)

in welcher
R' und $R^7$ die oben angegebene Bedeutung haben,
mit Basen gegebenenfalls in Gegenwart eines Verdünnungsmittels spaltet,
oder man erhält

h) die erfindungsgemäßen 5-Amino-1-phenyl-pyrazol-Derivate der Formel (Ih),

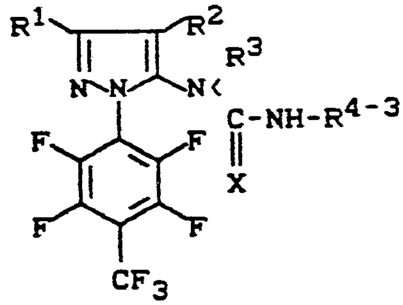

(Ih)

in welcher
$R^{4-3}$ für Alkyl oder für gegebenenfalls substituiertes Aryl steht und
$R^1$, $R^2$, $R^3$ und X die oben angegebene Bedeutung haben,
wenn man 5-Amino-1-phenyl-pyrazole der Formel (Is),

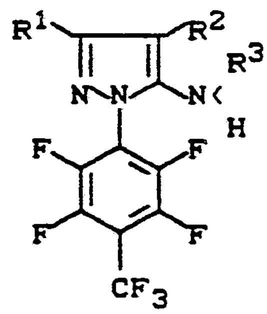

(Is)

in welcher $R^1$, $R^2$ und $R^3$ die oben angegebene Bedeutung haben,
mit Iso(thio)cyanaten der Formel (VII),
$R^{4-3}$-N=C=X (VII)
in welcher
$R^{4-3}$ und X die oben angegebene Bedeutung haben,
gegebenenfalls in Gegenwart eines Verdünnungsmittels und gegebenenfalls in Gegenwart eines Säurebindemittels umsetzt,
oder man erhält

i) die erfindungsgemäßen 5-Amino-1-phenyl-pyrazol-Derivate der Formel (Ii),

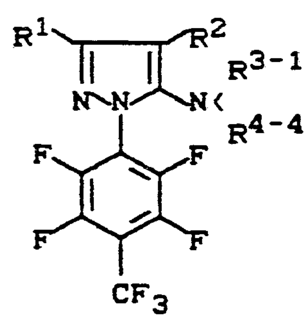

(Ii)

in welcher
$R^{4-4}$ für Alkyl steht,
$R^{3-1}$ für Wasserstoff oder Alkyl steht und
$R^1$ und $R^2$ die oben angegebene Bedeutung haben,
wenn man 5-Halogen-pyrazole der Formel (VIII),

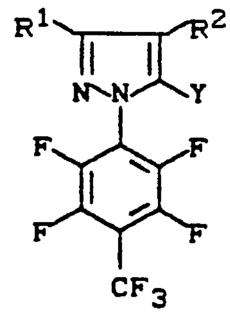

(VIII)

in welcher
Y für Halogen steht und
$R^1$ und $R^2$ die oben angegebene Bedeutung haben,
mit Aminen der Formel (IX),

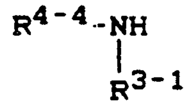

(IX)

in welcher
$R^{4-4}$ für Alkyl steht und
$R^{3-1}$ für Wasserstoff oder Alkyl steht,
gegebenenfalls in Gegenwart eines Verdünnungsmittels und gegebenenfalls in Gegenwart eines Säurebindemittels umsetzt,
oder man erhält

k) die erfindungsgemäßen 5-Amino-1-phenyl-pyrazol-Derivate der Formel (Ik),

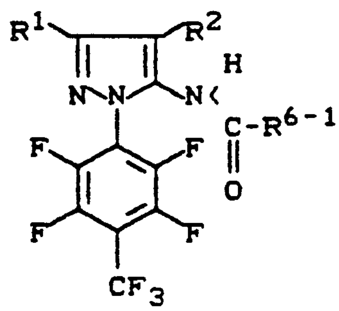

(Ik)

in welcher
$R^{6-1}$ für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht, und
$R^1$ und $R^2$ die oben angegebene Bedeutung haben,
wenn man (Bis)Carbamate der Formel (Iw),

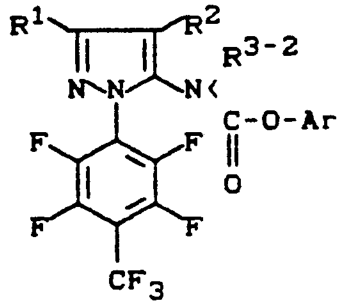

(Iw)

in welcher
$R^{3-2}$ für Wasserstoff oder für einen Rest
$-\overset{\overset{\displaystyle O}{\|}}{C}-O-Ar$ steht, wobei
Ar für gegebenenfalls substituiertes Aryl steht und
$R^1$ und $R^2$ die oben angegebene Bedeutung haben,
mit Verbindungen der Formel (X),
$R^{6-1}$ -H (X)
in welcher
$R^{6-1}$ die oben angegebene Bedeutung hat,
gegebenenfalls in Gegenwart eines Verdünnungsmittels und gegebenenfalls in Gegenwart eines basischen Katalysators umsetzt,
oder man erhält

l) Salze von erfindungsgemäßen 5-Sulfonamido-pyrazol-Derivaten der Formel (Ix),
wenn man 5-Sulfonamido-pyrazole der Formel (Ix),

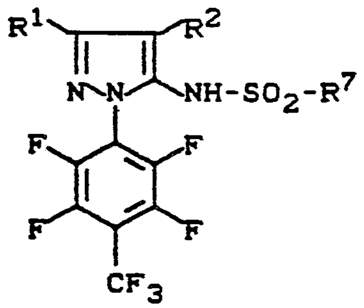

(Ix)

in welcher
$R^1$, $R^2$ und $R^7$ die oben angegebene Bedeutung haben,
entweder mit Salzen der Formel (XI),
$M^{\oplus}$ -$G^{\ominus}$ (XI)
in welcher
$M^{\oplus}$ für ein Äquivalent eines anorganischen oder organischen Kations steht und
$G^{\ominus}$ für ein Äquivalent eines geeigneten Gegenions steht,
oder mit primären, sekundären oder tertiären Aminen gegebenenfalls in Gegenwart eines Verdünnungsmittels umsetzt,
oder man erhält

m) Salze von erfindungsgemäßen 5-Perfluoracylamido-pyrazol-Derivaten der Formel (Iy),
wenn man 5-Perfluoracylamido-pyrazole der Formel (Iy),

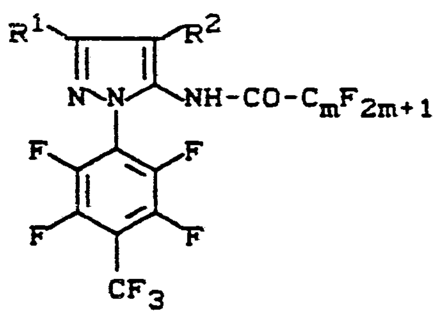

(Iy)

in welcher
$R^1$, $R^2$ und m die oben angegebene Bedeutung haben,
entweder mit Salzen der Formel (XI),
$M^{\oplus}$ -$G^{\ominus}$ (XI)
in welcher

$M^{\oplus}$ und $G^{\ominus}$ die oben angegebene Bedeutung haben, oder mit primären, sekundären oder tertiären Aminen gegebenenfalls in Gegenwart eines Verdünnungsmittels umsetzt.

Schließlich wurde gefunden, daß die neuen 5-Amino-1-phenyl-pyrazole der allgemeinen Formel (I) herbizide, insbesondere auch selektiv-herbizide und pflanzenwuchsregulierende Eigenschaften besitzen.

Überraschenderweise zeigen die erfindungsgemäßen 5-Amino-1-phenyl-pyrazole der allgemeinen Formel (I) neben einer deutlich verbesserten allgemein-herbiziden Wirksamkeit gegenüber Schadpflanzen auch eine erheblich verbesserte Verträglichkeit gegenüber wichtigen Kulturpflanzen im Vergleich zu den aus dem Stand der Technik bekannten 5-Amino-1-aryl-pyrazolen, wie beispielsweise das 4-Nitro-5-propion-amido-1-(2,3,5,6-tetrafluor-4-trifluormethylphenyl)-pyrazol, welche chemisch und wirkungsgemäß naheliegende Verbindungen sind. Darüber hinaus zeigen die erfindungsgemäßen 5-Amino-1-phenyl-pyrazole der Formel (I) überraschenderweise auch pflanzenwachstumsregulierende Eigenschaften.

Die erfindungsgemäßen 5-Amino-1-phenyl-pyrazole sind durch die Formel (I) allgemein definiert. Bevorzugt sind Verbindungen der Formel (I), bei welchen

$R^1$ für Wasserstoff oder für geradkettiges oder verzweigtes Alkyl mit 1 bis 8 Kohlenstoffatomen steht,

$R^2$ für Wasserstoff, Nitro, Nitroso, Fluor, Chlor, Brom, Iod oder für einen Rest $-\underset{\underset{O}{\|}}{C}-R^5$

steht,

wobei

$R^5$ für Wasserstoff, Hydroxy, geradkettiges oder verzweigtes Alkyl mit 1 bis 8 Kohlenstoffatomen, für jeweils geradkettiges oder verzweigtes Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten jeweils in Frage kommen: Halogen, jeweils geradkettiges oder verzweigtes Alkyl oder Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

$R^3$ für Wasserstoff, für einen Rest $-\underset{\underset{X}{\|}}{C}-R^6$

oder für einen Rest $-S(O)_n-R^7$ steht,

$R^4$ für Wasserstoff, für einen Rest $-\underset{\underset{X}{\|}}{C}-R^6$

oder für einen Rest $-S(O)_n-R^7$ steht, für geradkettiges oder verzweigtes Alkyl mit 1 bis 6 Kohlenstoffatomen steht, oder für den Fall, daß $R^3$ für einen Rest $-SO_2-R^7$ oder einen Rest $-CO-C_mF_{2m+1}$ steht auch für ein salzartig gebundenes Äquivalent eines Alkali-oder Erdalkali-oder Übergangsmetallkations oder für ein gegebenenfalls einfach oder mehrfach, gleich oder verschieden durch $C_1$-$C_6$-Alkyl, Phenyl und Benzyl substituiertes Ammoniumion steht, wobei

$R^6$ für Wasserstoff, für geradkettiges oder verzweigtes Alkyl mit 1 bis 12 Kohlenstoffatomen, für jeweils geradkettiges oder verzweigtes Alkenyl oder Alkinyl mit jeweils 2 bis 4 Kohlenstoffatomen, für jeweils geradkettiges oder verzweigtes Alkoxyalkyl, Alkylthioalkyl, Alkoxy, Alkylthio, Alkylsulfonylalkyl, Alkylsulfinylalkyl, Alkylamino, Dialkylamino oder Halogenalkyl mit jeweils 1 bis 4 Kohlenstoffatomen in den einzelnen Alkylteilen und im Fall des Halogenalkyl mit bis zu 9 gleichen oder verschiedenen Halogenatomen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten jeweils in Frage kommen: Halogen, jeweils geradkettiges oder verzweigtes Alkyl oder Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

$R^7$ für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder für gegebenenfalls einfach oder mehrfach, gleich oder verschieden substituiertes Phenyl steht, wobei als Phenylsubstituenten in Frage kommen: Halogen, jeweils geradkettiges oder verzweigtes Alkyl, Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

X für Sauerstoff oder Schwefel steht,

n für eine Zahl 0, 1 oder 2 steht und
m für eine Zahl 1, 2 oder 3 steht,
wobei jedoch für den Fall, daß $R^1$ und $R^3$ für Wasserstoff stehen und $R^2$ für Nitro steht, $R^4$ nicht gleichzeitig für einen Propionylrest steht.

Besonders bevorzugt sind Verbindungen der Formel (I), bei welchen
$R^1$ für Wasserstoff, Methyl, Ethyl, n-oder i-Propyl, n-, i-, s-oder t-Butyl steht,
$R^2$ für Wasserstoff, Nitro, Nitroso, Fluor, Chlor, Brom, Iod oder für einen Rest $-\underset{\underset{O}{\|}}{C}-R^5$
steht, wobei
$R^5$ für Wasserstoff, Hydroxy, Methyl, Ethyl, n-oder i-Propyl, n-, i-, s-oder t-Butyl, n-oder i-Pentyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 2-Chlorpropyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht,
$R^3$ für Wasserstoff, für einen Rest $-\underset{\underset{X}{\|}}{C}-R^6$
oder für einen Rest $-S(O)_n-R^7$ steht,
$R^4$ für Wasserstoff, für einen Rest
$-\overset{\overset{X}{\|}}{C}-R^6$ oder für einen Rest $-S(O)_n-R^7$ steht, für Methyl, Ethyl, n-oder i-Propyl, n-, i-, s-oder t-Butyl steht oder für den Fall, daß $R^3$ für Trifluormethylcarbonyl, Pentafluorethylcarbonyl, Heptafluor-n-propylcarbonyl, Heptafluor-i-propylcarbonyl oder für einen Rest $-SO_2-R^7$ steht, auch für ein salzartig gebundenes Äquivalent eines Natrium-, Kalium-, Magnesium-, Calcium-, Barium-, Kupfer-, Zink-, Mangan-, Zinn-, Eisen-, Cobalt- oder Nickelions steht, oder für ein gegebenenfalls ein-bis dreifach, gleich oder verschieden durch Methyl, Ethyl, n-oder i-Propyl, n-, i-, s-oder t-Butyl oder Phenyl substituiertes Ammoniumion steht, wobei
$R^6$ für Wasserstoff, Methyl, Ethyl, n-oder i-Propyl, n-, i-, s-oder t-Butyl, n-oder i-Pentyl, Hexyl, Heptyl, Octyl, Nonyl, Decyl, Undecyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 3-Chlorpropyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls einbis dreifach, gleich oder verschieden durch Methyl, Methoxy, Chlor oder Trifluormethyl substituiertes Phenyl, Phenoxy, Phenylthio oder Phenylamino steht,
$R^7$ für Methyl, Ethyl, n-oder i-Propyl, n-, i-, s-oder t-Butyl, Chlormethyl, Dichlormethyl, Trichlormethyl, Trifluormethyl oder für gegebenenfalls ein-bis dreifach, gleich oder verschieden durch Methyl, Methoxy, Chlor oder Trifluormethyl substituiertes Phenyl steht,
X für Sauerstoff oder Schwefel steht,
n für eine Zahl 0, 1 oder 2 steht,
wobei jedoch für den Fall, daß $R^1$ und $R^3$ für Wasserstoff stehen und $R^2$ für Nitro steht, $R^4$ nicht gleichzeitig für einen Propionylrest steht.

Eine ganz besonders bevorzugte Gruppe von Verbindungen der Formel (I) sind diejenigen, bei welchen
$R^1$ für Wasserstoff steht,
$R^2$ für Wasserstoff, Nitro, Nitroso, Fluor, Chlor, Brom, Iod oder für einen Rest $-\underset{\underset{O}{\|}}{C}-R^5$
steht, wobei
$R^5$ für Wasserstoff, Methyl, Ethyl, n-oder i-Propyl, n-, i-, s-oder t-Butyl, n-oder i-Pentyl, für gegebenenfalls ein-bis dreifach, gleich oder verschieden durch Methyl, Methoxy, Chlor oder Trifluormethyl substituiertes Phenyl steht,
$R^3$ für Wasserstoff oder für einen Rest $-\underset{\underset{X}{\|}}{C}-R^6$
steht,

$R^4$ für Wasserstoff, für einen Rest $-\overset{X}{\overset{\|}{C}}-R^6$ oder für Methyl, Ethyl, n-oder i-Propyl, n-, i-, s-oder t-Butyl steht,

$R^6$ für Wasserstoff, Methyl, Ethyl, n-oder i-Propyl, n-, i-, s-oder t-Butyl, n-oder i-Pentyl, Hexyl, Heptyl, Octyl, Nonyl, Decyl, Undecyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 3-Chlorpropyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht,

X für Sauerstoff oder Schwefel steht,

wobei jedoch die folgende Verbindung ausgeschlossen ist:

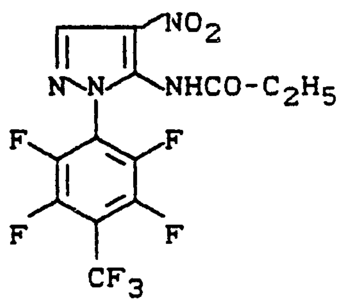

Eine andere ganz besonders bevorzugte Gruppe von Verbindungen der Formel (I) sind diejenigen, bei welchen

$R^1$ für Wasserstoff steht,

$R^2$ für Wasserstoff, Nitro, Nitroso, Fluor, Chlor, Brom, Iod oder für einen Rest $-\underset{O}{\underset{\|}{C}}-R^5$ steht, wobei

$R^5$ für Hydroxy, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 2-Chlorpropyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein-bis dreifach, gleich oder verschieden durch Methyl, Methoxy, Chlor oder Trifluormethyl substituiertes Phenoxy, Phenylthio oder Phenylamino steht,

$R^3$ für Wasserstoff, für einen Rest $-\underset{X}{\underset{\|}{C}}-R^6$ oder für einen Rest $-S(O)_n-R^7$ steht,

$R^4$ für Wasserstoff, für einen Rest $-\overset{X}{\overset{\|}{C}}-R^6$ oder für einen Rest $-S(O)_n-R^7$ steht, für Methyl, Ethyl, n-oder i-Propyl, n-, i-, s-oder t-Butyl steht oder für den Fall, daß $R^3$ für Trifluormethylcarbonyl, Pentafluorethylcarbonyl, Heptafluor-n-propylcarbonyl, Heptafluor-i-propylcarbonyl oder für einen Rest $-SO_2-R^7$ steht, auch für ein salzartig gebundenes Äquivalent eines Natrium-, Kalium-, Magnesium-, Calcium-, Barium-, Kupfer-, Zink-, Mangan-, Zinn-, Eisen-, Co-balt-oder Nickelions steht, oder für ein gegebenenfalls ein-bis dreifach, gleich oder verschieden durch Methyl, Ethyl, n-oder i-Propyl, n-, i-, s-oder t-Butyl oder Phenyl substituiertes Ammoniumion steht, wobei

$R^6$ für Wasserstoff, Methyl, Ethyl, n-oder i-Propyl, n-, i-, s-oder t-Butyl, n-oder i-Pentyl, Hexyl, Heptyl, Octyl, Nonyl, Decyl, Undecyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 3-Chlorpropyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht,

$R^7$ für Methyl, Ethyl, n-oder i-Propyl, n-, i-, s-oder t-Butyl, Chlormethyl, Dichlormethyl, Trichlormethyl, Trifluormethyl oder für gegebenenfalls ein-bis dreifach, gleich oder verschieden durch Methyl, Methoxy, Chlor oder Trifluormethyl substituiertes Phenyl steht,

X für Sauerstoff oder Schwefel steht,

n für eine Zahl 0, 1 oder 2 steht,

wobei jedoch für den Fall, daß $R^1$ und $R^3$ für Wasserstoff stehen und $R^2$ für Nitro steht, $R^4$ nicht gleichzeitig für einen Propionylrest steht.

Ganz besonders bevorzugt sind auch die Verbindungen der Formel (I), in denen

$R^1$ für Methyl, Ethyl, n-oder i-Propyl, n-, i-, s-oder t-Butyl steht und

$R^2$, $R^3$ und $R^4$ die oben als besonders bevorzugt angegebene Bedeutung haben.

Im einzelnen seien außer den bei den Herstellungsbeispielen genannten Verbindungen die folgenden 5-Amino-1-phenylpyrazole der allgemeinen Formel (I) genannt:

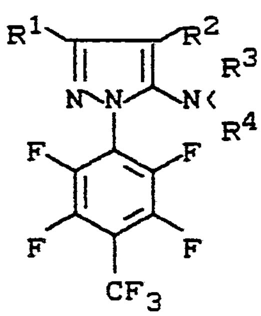

(I)

Tabelle 1

| $R^1$ | $R^2$ | $R^3$ | $R^4$ |
|---|---|---|---|
| H | $NO_2$ | H | $C_2H_5$ |
| H | $NO_2$ | H | -CO-O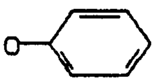 |
| H | $NO_2$ | -CO-O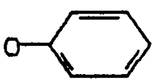 | -CO-O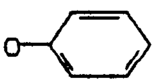 |
| $CH_3$ | $NO_2$ | H | H |
| $CH_3$ | $NO_2$ | H | $-COCH_3$ |
| H | $NO_2$ | H | $-COOCH_3$ |
| H | $NO_2$ | H | $-COCH_2Cl$ |
| H | $NO_2$ | H | $-COCH_2-O-C_2H_5$ |
| H | Cl | H | $-COC_2H_5$ |
| H | Br | H | $-COC_2H_5$ |
| H | Br | H | H |
| H | Cl | H | H |
| H | $NO_2$ | H | -CO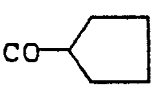 |
| H | $NO_2$ | H | $-COCH_2-SO_2CH_3$ |
| H | $NO_2$ | H | $-COCH(CH_3)_2$ |
| H | $NO_2$ | H | -COH |
| H | COH | H | $-COCH_3$ |
| H | COH | H | $-SO_2CH_3$ |

Tabelle 1 (Fortsetzung)

| $R^1$ | $R^2$ | $R^3$ | $R^4$ |
|---|---|---|---|
| H | $-CO-CH_3$ | H | H |
| H | NO | H | H |
| H | Cl | $-C_2H_5$ | $-C_2H_5$ |
| H | NO | H | $-C_3H_7$ |
| $CH_3$ | $NO_2$ | H | $-C_2H_5$ |

Verwendet man beispielsweise 2,3,5,6-Tetrafluor-4-trifluormethylphenyl-hydrazin und Ethoxymethylenmalonsäure-monoethylesternitril als Ausgangsstoffe, so läßt sich der Reaktionsablauf des erfindungsgemäßen Verfahrens (a) durch das folgende Formelschema darstellen:

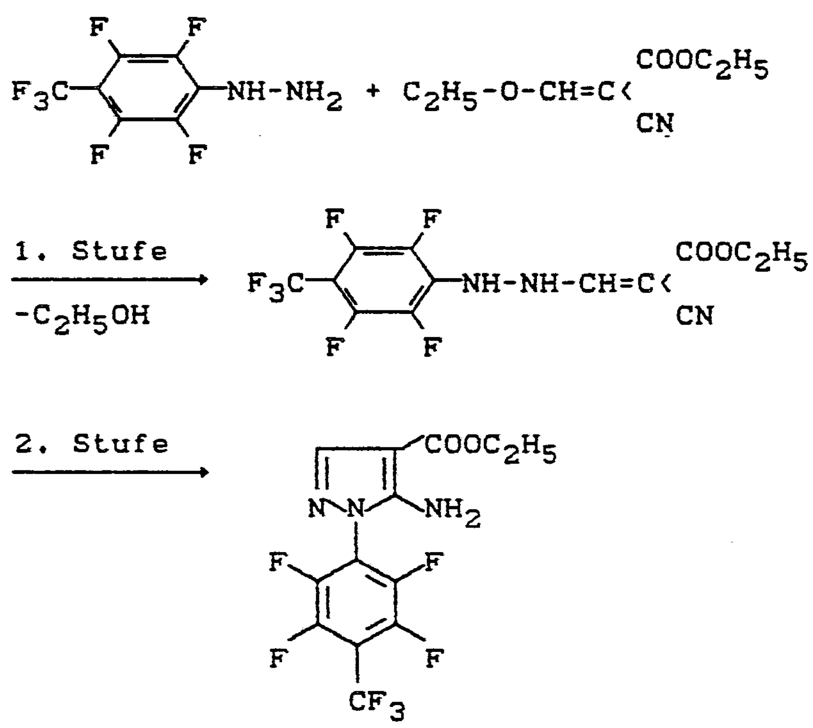


Verwendet man beispielsweise 5-Amino-1-(2,3,5,6-tetrafluor-4-trifluormethyl-phenyl)-4-ethoxycarbonyl-pyrazol als Ausgangsstoff, so läßt sich der Reaktionsablauf des erfindungsgemäßen Verfahrens (b) durch das folgende Formelschema darstellen:

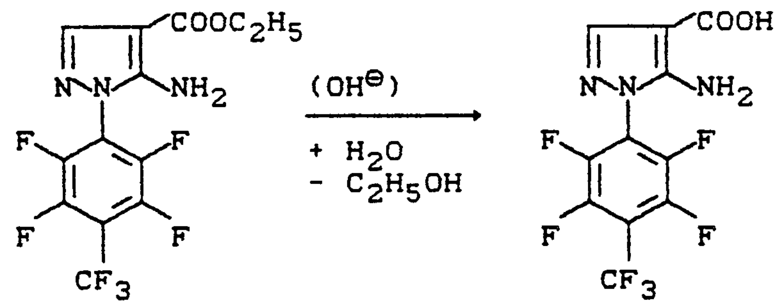


Verwendet man beispielsweise 5-Amino-1-(2,3,5,6-tetrafluor-4-trifluormethyl-phenyl)-pyrazol-4-carbonsäure als Ausgangsstoff, so läßt sich der Reaktionsablauf des erfindungsgemäßen Verfahrens (c) durch das folgende Formelschema darstellen:

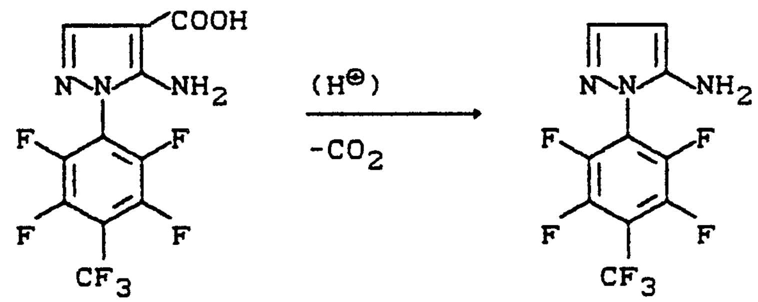


Verwendet man beispielsweise 5-Amino-1-(2,3,5,6-tetrafluor-4-trifluormethyl-phenyl)-pyrazol und Propionylchlorid bzw. Methylsulfonsäurechlorid bzw. Methyliodid als Ausgangsstoffe, so läßt sich der Reaktionsablauf des erfindungsgemäßen Verfahren (d-α), (d-β) und (d-γ) durch das folgende Formelschema darstellen:

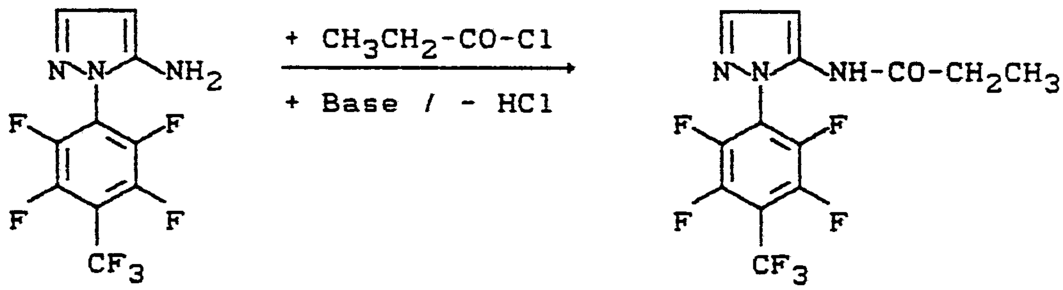


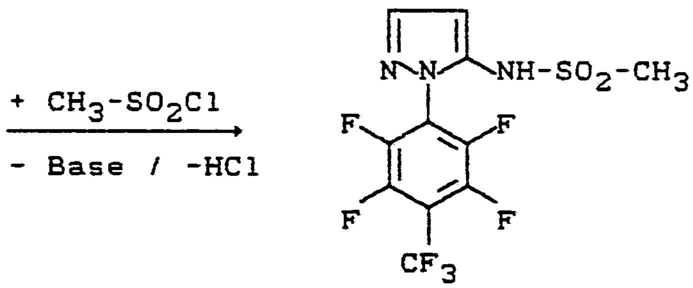


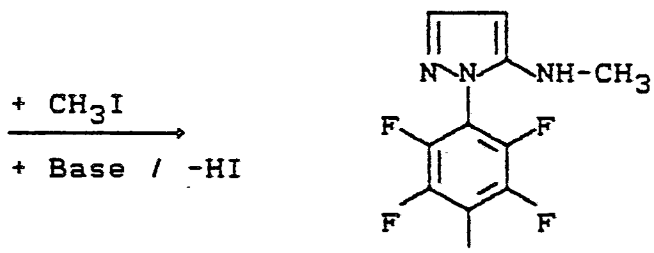


Verwendet man beispielsweise 1-(2,3,5,6-tetrafuor-4-trifluormethyl-phenyl-5-acetamido-pyrazol und Salpetersäure als Ausgangsstoffe, so läßt sich der Reaktionsablauf des erfindungsgemäßen Verfahrens (e) durch das folgende Formelschema darstellen:

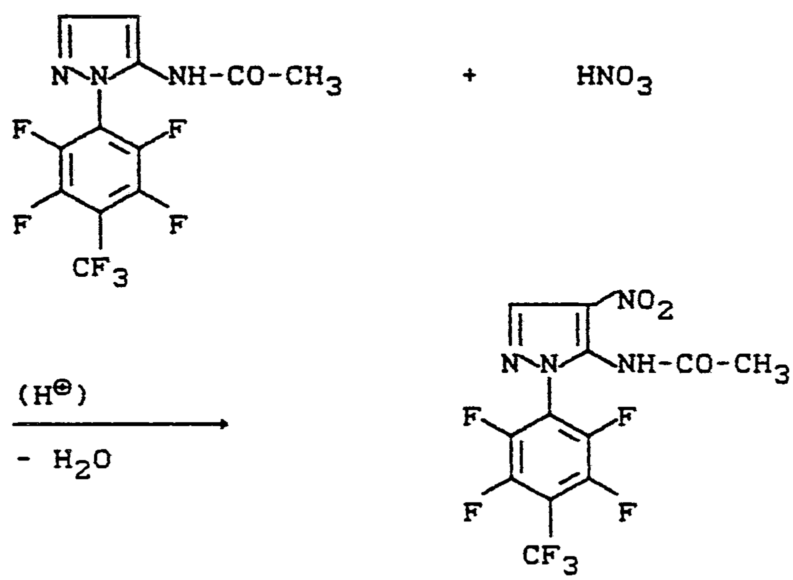


Verwendet man beispielsweise 1-(2,3,5,6-tetrafluor-4-trifluormethyl-phenyl)-4-nitro-5-acetamido-pyrazol als Ausgangsstoff, so läßt sich der Reaktionsablauf des erfindungsgemäßen Verfahrens (f) durch das folgende Formelschema darstellen:

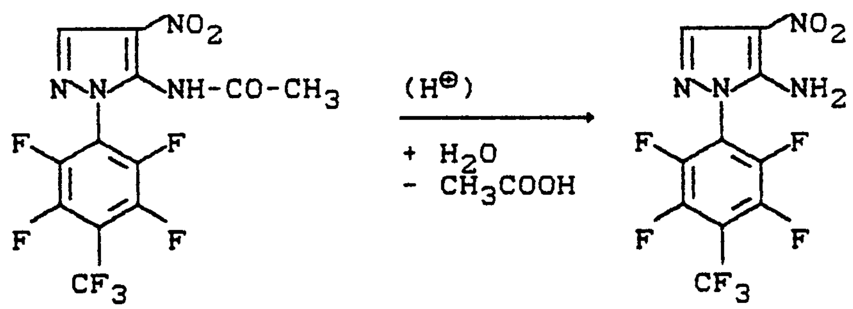


Verwendet man beispielsweise 5-[N,N-Bis(methansulfon)-amido]-1-(2,3,5,6-tetrafluor-4-trifluormethyl-phenyl)-pyrazol und Ammoniak als Ausgangsstoffe, so läßt sich der Reaktionsablauf des erfindungsgemäßen Verfahrens (g) durch das folgende Formelschema darstellen:

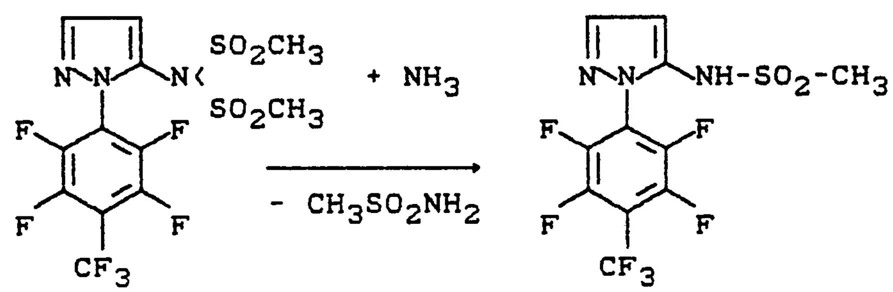


Verwendet man beispielsweise 5-Amino-1-(2,3,5,6-tetrafluor-4-trifluormethyl-phenyl)-pyrazol und Methylisocyanat als Ausgangsstoffe, so läßt sich der Reaktionsablauf des erfindungsgemäßen Verfahrens (h) durch folgendes Formelschema darstellen:

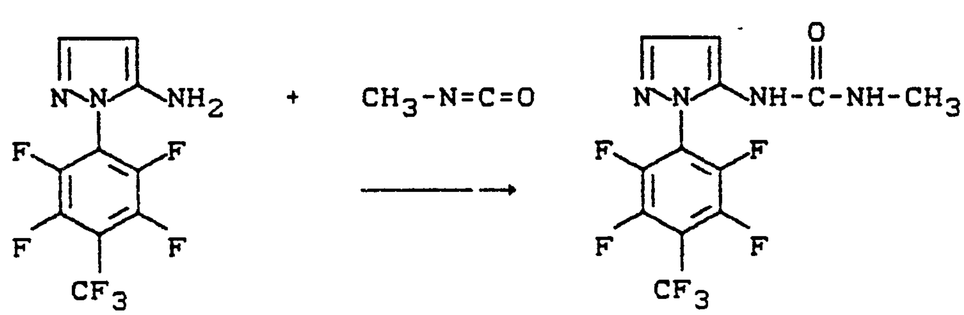


Verwendet man beispielsweise 5-Brom-4-nitro-1-(2,3,5,6-tetrafluor-4-trifluormethyl-phenyl)-pyrazol und Isopropylamin als Ausgangsstoffe, so läßt sich der Reaktionsablauf des erfindungsgemäßen Verfahrens (i) durch das folgende Formelschema darstellen:

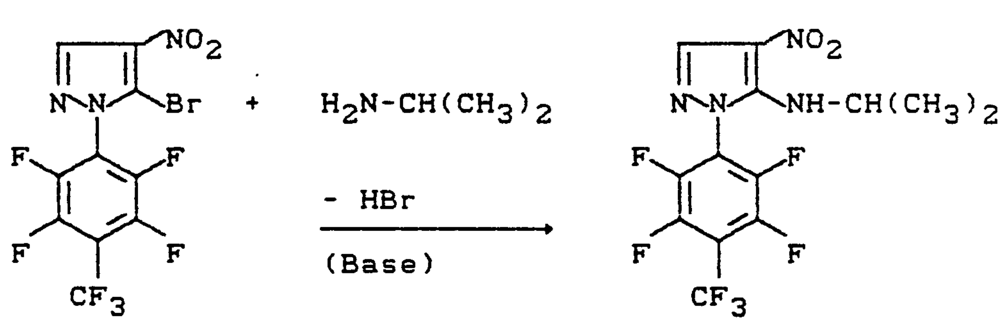

Verwendet man beispielsweise 5-Phenoxycarbonylamino-1-(2,3,5,6-tetrafluor-4-trifluormethyl-phenyl)-pyrazol und Methanol als Ausgangsstoffe, so läßt sich das erfindungsgemäße Verfahren (k) durch das folgende Formelschema darstellen:

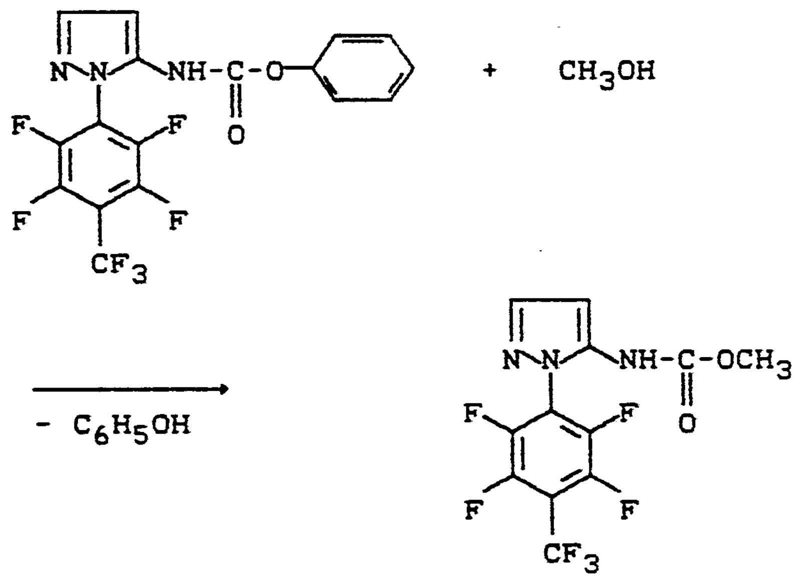


Verwendet man beispielsweise 1-(2,3,5,6-tetrafluor-4-trifluormethyl-phenyl)-5-methansulfonamido-pyrazol und Isopropylamin als Ausgangsstoffe, so läßt sich der Reaktionsablauf des erfindungsgemäßen Verfahrens (1) durch das folgende Formelschema darstellen:

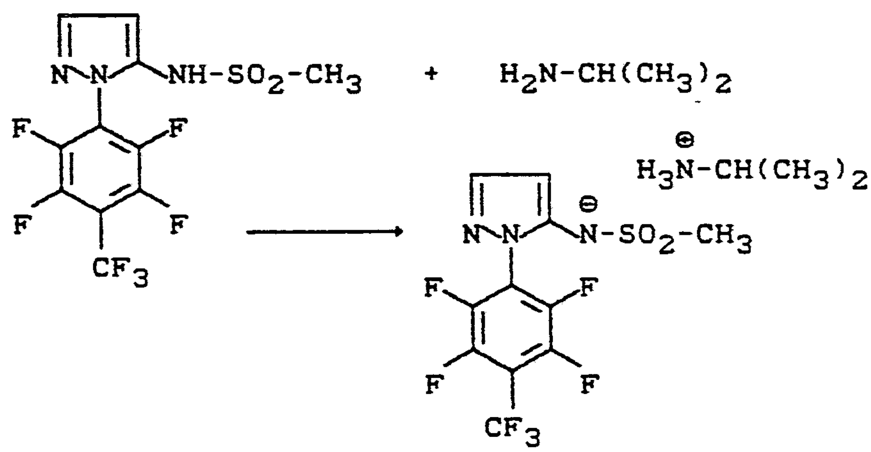

Verwendet man beispielsweise 1-(2,3,5,6-tetrafluor-4-trifluormethyl-phenyl)-5-trifluoracetamido-pyrazol und Natriumhydrogencarbonat als Ausgangsstoffe, so läßt sich der Reaktionsablauf des erfindungsgemäßen Verfahrens (m) durch das folgende Formelschema darstellen:

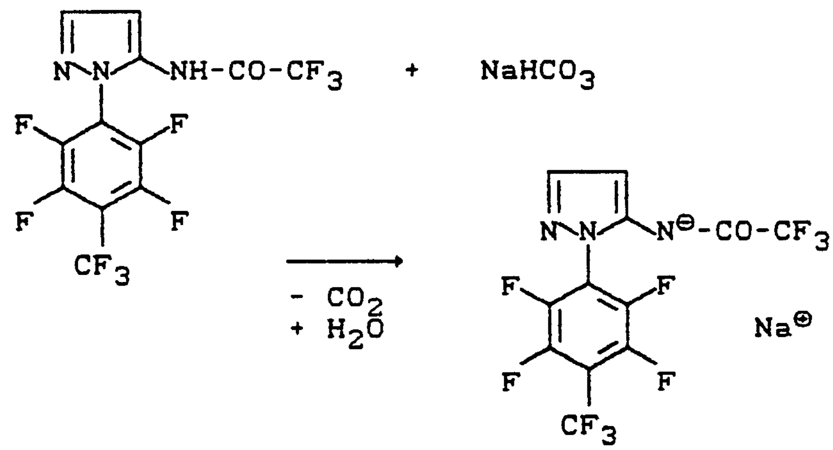


Das zur Durchführung des erfindungsgemäßen Verfahrens (a) als Ausgangsstoff benötigte 2,3,5,6-Tetrafluor-4-trifluormethylphenylhydrazin ist durch die Formel (II) definiert und bekannt (vergl. J. Chem. Soc. 1962, 1801, British UK Pat. Appl. GB 2 123 420 sowie PCT Int. Appl. WO 82/331).

Die zur Durchführung des erfindungsgemäßen Verfahrens (a) weiterhin als Ausgangsstoffe benötigten Acrylnitril-Derivate sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für $R^1$ und $R^2$ genannt wurden. A steht vorzugsweise für Chlor, Brom, Hydroxy, Methoxy, Ethoxy oder Dimethylamino.

Die Acrylnitril-Derivate der Formel (III) sind bekannt (vgl. DE-OS 31 29 429, DE-OS 32 06 878, EP 34 945; J.Chem. Soc.D. 1255; 1970, Can.J.Chem. 48. 2104-2109 (1970); J. Heterocyclic Chem. 19, 1267-1273 (1982); Can.J.Chem. 51, 1239-1244 (1973)) oder können nach bekannten Verfahren in einfacher analoger Weise erhalten werden.

Die zur Durchführung des erfindungsgemäßen Verfahrens (b) als Ausgangsstoffe benötigten 4-Alkoxycarbonyl-4-aminopyrazole sind durch die Formel (Ir) allgemein definiert. In der Formel (Ir) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden. $R^8$ steht vorzugsweise für geradkettiges oder verzweigtes Alkyl mit 1 bis 4 Kohlenstoffatomen, insbesondere für Methyl oder Ethyl.

Die 4-Alkoxycarbonyl-5-amino-pyrazole der Formel (Ir) sind erfindungsgemäße Verbindungen und erhältlich mit Hilfe des erfindungsgemäßen Verfahrens (a).

Die zur Durchführung des erfindungsgemäßen Verfahrens (c) als Ausgangsstoffe benötigten 5-Amino-1-phenyl-pyrazol-Derivate sind durch die Formel (Ib) allgemein definiert. In dieser Formel (Ib) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

Die 5-Amino-1-phenyl-pyrazol-Derivate der Formel (Ib) sind erfindungsgemäße Verbindungen und erhältlich mit Hilfe des erfindungsgemäßen Verfahrens (b).

Die zur Durchführung des erfindungsgemäßen Verfahrens (d) als Ausgangsstoffe benötigten 5-Amino-1-phenyl-pyrazole sind durch die Formel (Is) allgemein definiert. In dieser Formel (Is) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-phenyl-pyrazole der Formel (Is) sind erfindungsgemäße Verbindungen.

5-Amino-1-phenyl-pyrazole der Formel (Is), bei welchen $R^3$ für Wasserstoff steht, sind erhältlich mit Hilfe der erfindungsgemäßen Verfahren (a), (b), (c), (e) oder (f), 5-Amino-1-phenyl-pyrazole der Formel (Is), in welchen $R^3$ verschieden von Wasserstoff ist, sind erhältlich mit Hilfe der erfindungsgemäßen Verfahren - (e), (f), (g) oder (h).

Außerdem können 5-Amino-1-phenyl-pyrazole der Formel (Id), welche beispielsweise nach dem erfindungsgemäßen Verfahren (d-α) hergestellt werden, im erfindungsgemäßen Verfahren (d-γ) als Ausgangsstoffe eingesetzt werden.

Setzt man die mit Hilfe der erfindungsgemäßen Verfahren (d-α), (d-β) oder (d-γ) erhaltenen monoalkylierten, -acylierten, -sulfenylierten, -sulfinylierten oder -sulfonylierten Verbindungen erneut nach einem dieser Verfahren um, erhält man die entsprechenden disubstituierten Verbindungen.

Die zur Durchführung des erfindungsgemäßen Verfahrens (d) weiterhin benötigten Verbindungen sind durch die Formel (V), (Va) und (Vb) allgemein definiert. In den Formeln (V), (Va) und (Vb) stehen $R^{8-1}$ vorzugsweise für geradkettiges oder verzweigtes Alkyl mit 1 bis 6 Kohlenstoffatomen und $R^6$, $R^7$, X und n vorzugsweise für diejenigen Reste, die bereits im Zusammenhang mit der Beschreibung der erfindungsgemäßen Stoffe der Formel (I) als bevorzugt für diese Substituenten genannt wurden, $A^1$ steht vorzugsweise für Chlor oder Brom oder für einen

Rest $R^6-\overset{O}{\overset{\|}{C}}-O$, $A^2$ steht bevorzugt für Chlor oder Brom und $A^3$ steht bevorzugt für Chlor, Brom, Jod, p-Toluolsulfonyloxy oder Methoxysulfonyloxy.

Die Verbindungen der Formeln (V), (Va) und (Vb) sind allgemein bekannte Verbindungen der organischen Chemie.

Die zur Durchführung des erfindungsgemäßen Verfahrens (e) als Ausgangsstoffe benötigten 5-Amino-1-phenyl-pyrazole sind durch die Formel (It) allgemein definiert. In dieser Formel (It) stehen $R^1$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-phenyl-pyrazole der Formel (It) sind erfindungsgemäße Verbindungen und erhältlich mit Hilfe der erfindungsgemäßen Verfahren (a), (c), (d), (g), (i) oder (K).

Die zur Durchführung des erfindungsgemäßen Verfahrens (e) weiterhin als Ausgangsstoffe benötigten elektrophilen Agenzien sind durch die Formel (VI) allgemein definiert. In dieser Formel (VI) steht $R^{2-1}$ vorzugsweise für Chlor, Brom, Nitroso, Nitro, für Formyl, Alkanoyl mit 1 bis 6 Kohlenstoffatomen im Alkylteil oder für gegebenenfalls einfach oder mehrfach, gleich oder verschieden substituiertes Benzoyl, wobei als Substituenten in Frage kommen: Halogen, insbesondere Fluor, Chlor oder Brom, jeweils geradkettiges oder verzweigtes Alkyl, Alkoxy mit jeweils 1 bis 4 Kohlenstoffatomen, insbesondere für Methyl oder Methoxy, Halogenalkyl mit 1 bis 4 Kohlenstoffatomen und 1 bis 9 gleichen oder verschiedenen Halogenatomen, insbesondere Trifluormethyl.

$A^4$ steht vorzugsweise für Halogen, insbesondere für Chlor oder Brom, für Hydroxy, für Alkyl-oder Arylsulfonyloxy, für Alkanoyloxy oder Aroyloxy, Weiterhin verwendbare elektrophile Reagenzien sind Sulfurylchlorid, Phosphoroxychlorid/Diemthylformamid, Nitriersäure und andere üblicherweise zu elektrophilen Substitutionen verwendbare Stoffe.

Die elektrophilen Agenzien der Formel (VI) sind ebenso wie die weiteren üblichen elektrophilen Reagenzien allgemein bekannte Verbindungen.

Die zur Durchführung des erfindungsgemäßen Verfahrens (f) als Ausgangsstoffe benötigten 5-Acylamino-1-phenyl-pyrazole sind durch die Formel (Iu) allgemein definiert.

In dieser Formel (Iu) stehen $R^1$ und $R^6$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{2-2}$ steht vorzugsweise für Nitro, Nitroso, Fluor, Chlor, Brom oder Iod, $R^{4-2}$ steht vorzugsweise für Wasserstoff oder für geradkettiges oder verzweigtes Alkyl mit 1 bis 4 Kohlenstoffatomen, insbesondere für Methyl oder Ethyl.

Die 5-Acylamino-1-phenyl-pyrazole der Formel (Iu) sind erfindungsgemäße Verbindungen und erhältlich mit Hilfe der erfindungsgemäßen Verfahren (d) oder (e).

Die zur Durchführung des erfindungsgemäßen Verfahrens (g) als Ausgangsstoffe benötigten 5-Bis-sulfonyl-amino-pyrazole sind durch die Formel (Iv) allgemein definiert. In dieser Formel (Iv) stehen $R^1$ und $R^7$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Bis-sulfonyl-amino-pyrazole der Formel (Iv) sind erfindungsgemäße Verbindungen und erhältlich mit Hilfe des erfindungsgemäßen Verfahrens (d).

Die zur Durchführung des erfindungsgemäßen Verfahrens (h) als Ausgangsstoffe benötigten 5-Amino-1-phenyl-pyrazole sind durch die Formel (Is) allgemein definiert.

In dieser Formel (Is) stehen $R^1$, $R^2$ und $R^3$ vorzugsweise für diejenigen Bedeutungen, die bereits im Zusammenhang mit der Beschreibung der erfindungsgemäßen Stoffe der Formel (I) als bevorzugt für diese Substituenten genannt wurden.

Die 5-Amino-1-phenyl-pyrazole der Formel (Is) sind erfindungsgemäße Verbindungen. Verbindungen der Formel (Is), bei welchen $R^3$ für Wasserstoff steht, sind erhältlich mit Hilfe der erfindungsgemäßen Verfahren (a), (b), (c), (e) oder (f).

Verbindungen der Formel (Is), in welchen $R^3$ verschieden von Wasserstoff ist, sind erhältlich mit Hilfe der erfindungsgemäßen Verfahren (d), (e), (f), (g) oder (h).

Die zur Durchführung des erfindungsgemäßen Verfahrens (h) weiterhin als Ausgangsstoffe benötigten Iso(thio)-cyanate sind durch die Formel (VII) allgemein definiert. In dieser Formel (VII) steht X für Sauerstoff oder Schwefel und $R^{4-3}$ steht vorzugsweise für geradkettiges oder verzweigtes Alkyl mit 1 bis 4 Kohlenstoffatomen oder für gegebenenfalls ein-bis dreifach, gleich oder verschieden substituiertes Phenyl, wobei als Substituenten in Frage kommen: Halogen, jeweils geradkettiges oder verzweigtes Alkyl oder Alkoxy mit jeweils 1 bis 4 Kohlenstoffatomen oder Halogenalkyl mit 1 bis 4 Kohlenstoffatomen und 1 bis 9 gleichen oder verschiedenen Halogenatomen. $R^{4-3}$ steht insbesondere für Methyl, Ethyl oder für gegebenenfalls ein- bis dreifach, gleich oder verschieden durch Fluor, Chlor, Methyl, Methoxy oder Trifluormethyl substituiertes Phenyl.

Die Iso(thio)cyanate der Formel (VII) sind allgemein bekannte Verbindungen der organischen Chemie.

Die zur Durchführung des erfindungsgemäßen Verfahrens (i) als Ausgangsstoffe benötigten 5-Halogen-pyrazole sind durch die Formel (VIII), allgemein definiert. In dieser Formel (VIII) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden, Y steht vorzugsweise für Chlor oder Brom.

Die 5-Halogen-pyrazole der Formel (VIII) sind noch nicht bekannt. Sie sind jedoch großenteils Gegenstand der eigenen vorgängigen noch nicht veröffentlichten Patentanmeldungen DE-P 35 01 323 vom 17.01.1985 und DE-P 35 20 329 vom 07.06.1985.

Man erhält sie beispielsweise, wenn man Alkoxymethylenmalonester der Formel (XII),

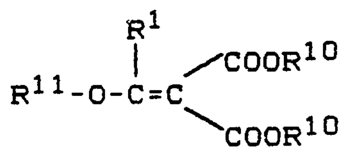

(XII)

in welcher

$R^1$ die oben angegebene Bedeutung hat und

$R^{10}$ und $R^{11}$ unabhängig voneinander jeweils für Alkyl, insbesondere für Methyl oder Ethyl stehen,

mit 2,3,5,6-Tetrafluor-4-trifluormethylphenyl-hydrazin der Formel (II)

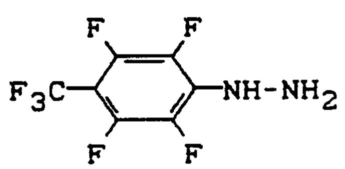

(II)

zunächst in einer ersten Stufe gegebenenfalls in Gegenwart eines Verdünnungsmittels wie beispielsweise Methanol oder Ethanol bei Temperaturen zwischen +10°C und +80°C umsetzt, und die so erhältlichen Pyrazolcarbonsäureester der Formel (XIII)

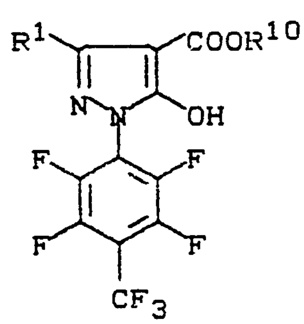

(XIII)

in welcher

$R^1$ und $R^{10}$ die oben angegebene Bedeutung haben,

in einer 2. Stufe gegebenenfalls in Gegenwart eines Verdünnungsmittels wie beispielsweise Methanol und gegebenenfalls in Gegenwart einer Base wie Beispielsweise Natriumhydroxid bei Temperaturen zwischen

+30°C und +70°C decarboxyliert zu Pyrazolinonen der Formel (XIV),

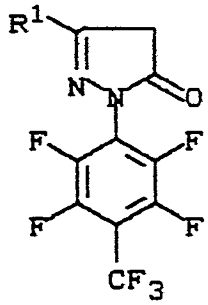

(XIV)

in welcher
$R^1$ die oben angegebene Bedeutung hat,
und diese in einer 3. Stufe mit Halogenierungsmitteln, wie beispielsweise Phosphoroxychlorid oder Phosphoroxybromid, nach üblichen bekannten Verfahren (vgl. z.B Ber. dtsch. chem. Ges. 28, 35 (1895) oder Liebigs Ann. Chem. 373 , 129 (1910)) umsetzt, und gegebenenfalls in einer 4. Stufe die so erhältlichen 5-Halogen-pyrazole der Formel (VIIIa),

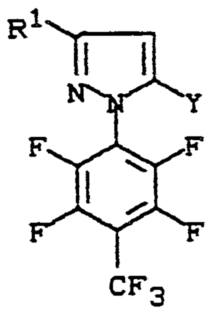

(VIIIa)

in welcher
$R^1$ und Y die oben angegebene Bedeutung haben,
in allgemein üblicher Art und Weise mit elektrophilen Agenzien der Formel (VI)
$R^{2-1}$-$A^4$ (VI)
in welcher
$R^{2-1}$ für Halogen, Nitroso, Nitro, Formyl, Alkanoyl oder Aroyl steht und
$A^4$ für eine elektronenanziehende Abgangsgruppe steht,
oder mit anderen üblichen elektrophilen Reagenzien gegebenenfalls in Gegenwart eines Verdünnungsmittels, wie beispielsweise Eisessig, und gegebenenfalls in Gegenwart eines Katalysators oder Reaktionshilfsmittels, wie beispielsweise Acetanhydrid, in Analogie zur Durchführung des erfindungsgemäßen Verfahrens (e) in 4-Stellung substituiert.

Die bei der Umsetzung von Alkoxymethylenmalonestern der Formel (XII) mit 2,3,5,6-Tetrafluor-4-trifluormethylphenyl-hydrazin der Formel (II) auftretenden Zwischenprodukte der Formel (XIIa),

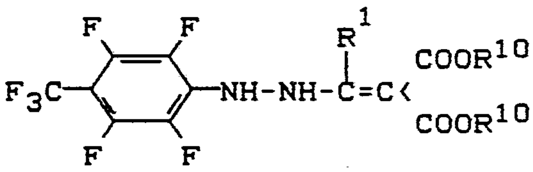

(XIIa)

in welcher
$R^1$ und $R^{10}$ die oben angegebene Bedeutung haben,
können gegebenenfalls auch isoliert und in einer separaten Reaktionsstufe cyclisiert werden.

Die Cyclisierung zu den Pyrazolcarbonsäureestern der Formel (XIII) und deren anschließende Decarboxylierung können gegebenenfalls in einer Reaktionsstufe als "Eintopfverfahren" durchgeführt werden (vgl. z.B. Liebigs Ann. Chem. 373, 142 (1910)).

Die Alkoxymethylenmalonester der Formel (XII) sind allgemein bekannte Verbindungen der organischen Chemie.

Die zur Durchführung des erfindungsgemäßen Verfahrens (i) weiterhin als Ausgangsstoffe benötigten Amine sind durch die Formel (IX) allgemein definiert. In dieser formel (IX) steht $R^{4-4}$ vorzugsweise für geradkettiges oder verzweigtes Alkyl mit 1 bis 6 Kohlenstoffatomen, insbesondere für Methyl, Ethyl, n-oder i-Propyl oder n-, i-, s-oder t-Butyl, $R^{3-1}$ steht vorzugsweise für Wasserstoff oder für geradkettiges oder verzweigtes Alkyl mit 1 bis 6 Kohlenstoffatomen, insbesondere für Methyl, Ethyl, n-oder i-Propyl oder n-, i-, s-oder t-Butyl.

Die Amine der Formel (IX) sind ebenfalls allgemein bekannte Verbindungen der organischen Chemie.

Die zur Durchführung des erfindungsgemäßen Verfahrens (k) als Ausgangsstoffe benötigten (Bis)-Carbamate sind durch die Formel (Iw) allgemein definiert. In dieser Formel (Iw) stehen $R^1$ und $R^2$ vorzugsweise für diejenigen Reste, die bereits im Zusammenhang mit der Beschreibung der erfindungsgemäßen Stoffe der Formel (I) als bevor zugt für diese Substituenten genannt wurden, $R^{3-2}$ steht für einen Rest -CO-O-Ar oder für Wasserstoff, wobei Ar vorzugsweise für Phenyl steht.

Die (Bis)Carbamate der Formel (Iw) sind erfindungsgemäße Verbindungen und erhältlich mit Hilfe der erfindungsgemäßen Verfahren (d) oder (e).

Die zur Durchführung des erfindungsgemäßen Verfahrens (k) weiterhin als Ausgangsstoffe benötigten Verbindungen sind durch die Formel (X) allgemein definiert. In dieser Formel (X) steht $R^{6-1}$ vorzugsweise für jeweils geradkettiges oder verzweigtes Alkoxy, Alkylthio, Alkylamino oder Dialkylamino mit jeweils 1 bis 4 Kohlenstoffatomen in den einzelnen Alkylteilen, für jeweils einfach oder mehrfach, gleich oder verchieden substituiertes Phenoxy, Phenylthio oder Phenylamino, wobei als Phenylsubstituenten jeweils in Frage kommen: Halogen, jeweils geradkettiges oder verzweigtes Alkyl oder Alkoxy mit jeweils 1 bis 4 Kohlenstoffatomen, Halogenalkyl mit 1 bis 4 Kohlenstoffatomen und 1 bis 9 gleichen oder verschiedenen Halogenatomen, insbesondere Methyl, Methoxy, Chlor oder Trifluormethyl, $R^{6-1}$ steht insbesondere für Methoxy, Ethoxy, Methylthio, Phenylthio oder Dimethylamino.

Die Verbindungen der Formel (X) sind allgemein bekannte Verbindungen der organischen Chemie.

Die zur Durchführung des erfindungsgemäßen Verfahrens (1) als Augangsstoffe benötigten 5-Sulfonamido-pyrazole sind durch die Formel (Ix) allgemein definiert. In dieser Formel (Ix) stehen $R^1$, $R^2$ und $R^7$ vorzugsweise für diejenigen Reste, die bereits im Zusammenhang mit der Beschreibung der erfindungsgemäßen Stoffe der Formel (I) als bevorzugt für diese Substituenten genannt wurden. Die 5-Sulfonamido-pyrazole der Formel (Ix) sind erfindungsgemäße Verbindungen und erhältlich mit Hilfe der erfindungsgemäßen Verfahren (d), (e) und (g).

Die zur Durchführung des erfindungsgemäßen Verfahrens (m) als Ausgangsstoffe benötigten 5-Perfluoracylamido-pyrazole sind durch die Formel (Iy) allgemein definiert. In dieser Formel (Iy) stehen $R^1$, $R^2$ und m vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Perfluoracylamido-pyrazole der Formel (Iy) sind erfindungsgemäße Verbindungen und erhältlich mit Hilfe der erfindungsgemäßen Verfahren (d) und (e).

Die zur Durchführung der erfindungsgemäßen Verfahren (1) und (m) weiterhin als Ausgangsstoffe benötigten Salze sind durch die Formel (XI) allgemein definiert. Vorzugsweise verwendet man Alkali-, Erdalkali-, Ammonium-oder Übergangsmetallhydroxide, -oxide, -carbonate, -hydrogencarbonate oder leicht lösliche -chloride, -sulfate, -phosphate oder -nitrate, wie beispielsweise Natrium-, Kalium-, Calciumhydroxid, -carbonat oder -hydrogencarbonat, Calciumchlorid, Bariumchlorid, Kupfersulfat, Nickelchlorid oder Cobaltnitrat oder Alkylamine, wie Triethylamin, Iso propylamin, Diisopropylamin, Butylamin.

Die Salze der Formel (XI) sind allgemein bekannte Verbindungen.

Als Verdünnungsmittel zur Durchführung des Herstellungsverfahrens (a) kommen sowohl für die 1. als auch für die 2. Reaktionsstufe inerte organische Lösungsmittel in Frage. Vorzugsweise verwendet man Alkohole, wie Methanol, Ethanol, Propanol, Butanol, Ethylenglykol oder Ethylenglykolmonomethyl-oder -ethylether.

Als Reaktionshilfsmittel zur Durchführung der 1. Stufe des Herstellungsverfahrens (a) kommen organische oder anorganische Säuren in Frage. Vorzugsweise verwendet man Schwefelsäure oder Essigsäure, gegebenenfalls auch in Gegenwart einer Puffersubstanz, wie beispielsweise Natriumacetat.

Die Reaktionstemperaturen können bei der Durchführung der 1. Stufe des Herstellungsverfahrens (a) in gewissen Bereichen variiert werden. Im allgemeinen arbeitet man zwischen -30 °C und +50 °C, vorzugsweise zwischen -20 °C und +20 °C.

Als Säurebindemittel zur Durchführung der 2. Stufe des Herstellungsverfahrens (a) kommen alle üblicherweise verwendbaren anorganischen und organischen Basen in Frage. Vorzugsweise verwendet man Alkalimetallcarbonate oder Hydrogencarbonate, wie Natriumcarbonat, Kaliumcarbonat oder Kaliumhydrogencarbonat.

Das erfindungsgemäße Verfahren (a) kann auch direkt in einem Reaktionsschritt ohne Isolierung der Zwischenprodukte der Formel (IV) durchgeführt werden.

Die Reaktionstemperaturen können bei der Durchführung der 2. Stufe des Herstellungsverfahrens (a) ebenso wie bei der einstufigen Reaktionsführung i einem größeren Bereich variiert werden. Im allgemeinen arbeitet man zwischen 0 °C und 200 °C, vorzugsweise zwischen +50 °C und +150 °C.

Zur Durchführung des Herstellungsverfahrens (a) setzt man sowohl bei der einstufigen als auch bei der zweistufigen Reaktionsführung pro Mol 2,3,5,6-Tetrafluor-4-trifluormethylphenyl-hydrazin der Formel (II) im allgemeinen 1,0 bis 3,0 Mol, vorzugsweise 1,0 bis 1,5 Mol an Acrylnitril-Derivat der Formel (III) und im Fall des zweistufigen Verfahrens gegebenenfalls in der 1. Stufe 1,0 bis 10,0 Mol an Reaktionshilfsmittel und gegebenenfalls in der 2. Stufe 1,0 bis 10,0 Mol an Säurebindemittel ein.

Die Aufarbeitung und Isolierung der Reaktionsprodukte der Formel (Ia) erfolgt nach üblichen Verfahren, beispielsweise durch Entfernen des organischen Verdünnungsmittels, Ausfällen des Reaktionsproduktes in Wasser, Absaugen und Trocknen des so erhaltenen Produktes.

Als Verdünnungsmittel zur Durchführung des Herstellungsverfahrens (b) kommen anorganische oder organische Lösungsmittel in Frage, Vorzugsweise verwendet man polare Lösungsmittel, insbesondere Alkohole, wie beispielsweise Methanol, Ethanol oder Propanol, oder deren Gemische mit Wasser.

Als Katalysatoren zur Durchführung des Herstellungsverfahrens (b) kommen alle üblicherweise für derartige Esterverseifungen verwendbaren Katalysatoren in Frage. Vorzugsweise verwendet man Basen, wie beispielsweise Natriumhydroxid, Natriumalkoholat oder Natriumcarbonat oder Säuren, wie beispielsweise Chlorwasserstoffsäure, Bromwasserstoffsäure oder Schwefelsäure.

Die Reaktionstemperaturen können bei der Durchführung des erfindungsgemäßen Verfahrens (b) in einem größeren Bereich vriiert werden. Im allgemeinen arbeitet man bei Temperaturen zwischen 20 °C und 150 °C, vorzugsweise bei Temperaturen zwischen 50 °C und 100 °C.

Zur Durchführung des Herstellungsverfahrens (b) setzt man pro Mol an 4-Alkoxycarbonyl-5-aminopyrazol der Formel (Ir) im allgemeinen 1,0 bis 15,0 Mol, vorzugsweise 1,0 bis 2,5 Mol an saurem oder basischem Katalysator ein und erwärmt für mehrere Stunden auf die erforderliche Reaktionstemperatur. Die Aufarbeitung, Isolierung und Reinigung der Reaktionsprodukte der Formel (Ib) erfolgt nach üblichen Verfahren.

Als Verdünnungsmittel zur Durchführung des Herstellungsverfahrens (c) kommen ebenfalls anorganische oder organische, vorzugsweise polare Lösungsmittel in Frage.

Insbesondere sind Alkohole, wie beispielsweise Methanol, Ethanol oder Propanol, oder deren Gemische mit Wasser geeignet.

Als Katalysatoren zur Duchführung des Herstellungsverfahrens (c) kommen vorzugsweise Säuren, insbesondere anorganische Mineralsäuren wie Chlorwasserstoffsäure, Bromwasserstoffsäure oder Schwefelsäure in Frage.

Die Reaktionstemperaturen können bei der Durchführung des Herstellungsverfahrens (c) in einem größeren Bereich variiert werden. Im allgemeinen arbeitet man zwischen +50 °C und +200 °C, vorzugsweise zwischen +70 °C und +130 °C.

Bei der Durchführung des erfindungsgemäßen Verfahrens (c) setzt man pro Mol an 5-Amino-1-phenylpyrazol-Derivat der Formel (Ib) im allgemeinen 1,0 bis 30,0 Mol, vorzugsweise 1,0 bis 15,0 Mol an Katalysatorsäure ein und erwärmt für mehrere Stunden auf die erforderliche Temperatur. Die Aufarbeitung, Isolierung und Reinigung der Reaktionsprodukte der Formel (Ic) erfolgt nach allgemein üblichen Verfahren.

Bei Verwendung eines sauren Katalysators ist es auch möglich die erfindungsgemäßen Verfahren (b) - (Esterverseifung) und (c) (Decarboxylierung) in einem Reaktionsschritt als Eintopfverfahren durchzuführen. Auch in diesem Fall erfolgt die Reaktionsdurchführung, Aufarbeitung und Isolierung der Reaktionsprodukte nach allgemein üblichen Methoden (vergl. auch Herstellungsbeispiele).

Zur Durchführung des erfindungsgemäßen Verfahrens (d) kommen als Verdünnungsmittel inerte organische Lösungsmittel infrage. Vorzugsweise verwendet man aliphatische, cyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid. Verwendet man Verbindungen der Formeln (V), (Va) oder (Vb) in flüssiger Form, so ist es auch möglich, diese in entsprechendem Überschuß als Verdünnungsmittel einzusetzen.

Als Säurebindemittel zur Durchführung des erfindungsgemäßen Verfahrens (d) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (d) in einem größeren Bereich variiert werden. Im allgemeinen arbeitet man zwischen -20 °C und +150 °C, vorzugsweise zwischen 0 °C und +100 °C.

Zur Durchführung des Herstellungsverfahrens (d) setzt man pro Mol 5-Amino-1-phenyl-pyrazol der Formel (Is) im allgemeinen 1,0 bis 20,0 Mol, vorzugsweise 1,0 bis 15,0 Mol an Verbindung der Formel (V), -(Va) bzw. (Vb) und gegebenenfalls 1,0 bis 3,0 Mol, vorzugsweise 1,0 bis 2,0 Mol an Säurebindemittel ein. Die Reaktionsdurchführung, Aufarbeitung und Isolierung der Reaktionsprodukte der Formel (Id) erfolgt in allgemein üblicher Art und Weise.

Als Verdünnungsmittel zur Durchführung des erfindungsgemäßen Verfahrens (e) kommen alle üblicherweise für derartige elektrophile Substitutionenn verwendbaren Lösungsmittel in Frage. Vorzugsweise verwendet man die als Reagenzien in Frage kommenden Säuren oder Gemische, wie beispielsweise Schwefelsäure, Salpetersäure, Sulfurylchlorid, Phosphoroxychlorid/Dimethylformamid oder Nitriersäure, gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff, als Verdünnungsmittel in Frage.

Als Katalysatoren oder Reaktionshilfsmittel zur Durchführung des Herstellungsverfahrens (e) kommen ebenfalls die für derartige Reakionen üblichen Katalysatoren in Frage; vorzugsweise verwendet man saure Katalysatoren wie beispielsweise Schwefelsäure, Eisen-III-chlorid oder andere Lewis-Säuren oder Acetanhydrid.

Die Reaktionstemperaturen können bei der Durchführung des Herstellungsverfahrens (e) in einem größeren Bereich variiert werden. Im allgemeinen arbeitet man zwischen -50 °C und +200 °C, vorzugsweise zwischen -20 °C und +150 °C.

Zur Durchführung des Herstellungsverfahrens (e) setzt man pro Mol 5-Amino-1-phenyl-pyrazol der Formel (It), im allgemeinen 1,0 bis 10,0 Mol, vorzugsweise 1,0 bis 5,0 Mol an elektrophilem Agens der Formel (VI) und gegebenenfalls 0,1 bis 10 Mol an Katalysator oder Reaktionshilfsmittel ein. Die Reaktionsführung, Aufarbeitung und Isolierung der Reaktionsprodukte der Formel (Ie) erfolgt in allgemein üblicher Art und Weise.

Als Verdünnungsmittel zur Durchführung des erfindungsgemäßen Verfahrens (f) kommen anorganische oder organische polare Lösungsmittel in Frage. Vorzugsweise verwendet man Alkohole, wie beispielsweise Methanol, Ethanol oder Propanol, oder deren Gemische mit Wasser.

Als Katalysatoren zur Durchführung des Herstellungsverfahrens (f) kommen vorzugsweise Säuren, insbesondere Chlorwasserstoffsäure oder Schwefelsäure in Frage.

Die Reaktionstemperaturen können bei der Durchführung des Herstellungsverfahrens (f) in einem größeren Bereich variiert werden. Im allgemeinen arbeitet man zwischen +20 °C und +150 °C, vorzugsweise zwischen +50 °C und +120 °C.

Zur Durchführung des Herstellungsverfahrens (f) setzt man pro Mol 5-Acylamino-1-phenyl-pyrazol der Formel (Iu) im allgemeinen 1,0 bis 20,0 Mol, vorzugsweise 1,0 bis 10,0 Mol an Katalysatorsäure ein und erwärmt für mehrere Stunden auf die erforderliche Reaktionstemperatur. Die Aufarbeitung, Isolierung und Reinigung der Reaktionsprodukte der Formel (If) erfolgt nach üblichen Methoden.

Als Verdünnungsmittel zur Durchführung des erfindungsgemäßen Verfahrens (g) kommen polare organische Lösungsmittel oder deren Gemische mit Wasser in Frage. Vorzugsweise verwendet man Alkohole wie Methanol, Ethanol oder Propanol oder deren Gemische mit Wasser.

Als basische Reaktionsteilnehmer bei der Durchführung des erfindungsgemäßen Verfahrens (g) kommen alle üblichen anorganischen oder organischen Basen in Frage, Vorzugsweise verwendet man Amine oder Ammoniaklösungen oder Alkalimetallcarbonate bzw. -hydrogencarbonate, wie Natrium-oder Kaliumcarbonat oder Natriumhydrogencarbonat.

Die Reaktionstemperaturen können bei der Durchführung des erfindungsgemäßen Verfahrens (g) in einem größeren Bereich variiert werden. Im allgemeinen arbeitet man zwischen 0 °C und 80 °C, vorzugsweise zwischen 10 °C und 40 °C.

Zur Durchführung des erfindungsgemäßen Verfahrens (g) setzt man pro Mol 5-Bis-sulfonyl-amino-pyrazol der Formel (Iv) im allgemeinen 1,0 bis 30,0 Mol vorzugsweise 1,0 bis 15,0 Mol an Base ein.

Die Reaktionsmischung wird in einem geeigneten Verdün nungsmittel so lange gerührt (30 Minuten bis 20 Stunden) bis bei chromatographischer Kontrolle kein Ausgangsprodukt mehr nachweisbar ist. Die Aufarbeitung der Reaktionsprodukte der Formel (Ig) erfolgt nach üblichen Methoden.

Zur Durchführung des erfindungsgemäßen Verfahrens (h) kommen als Verdünnungsmittel inerte organische Lösungsmittel in Frage. Vorzugsweise verwendet man die bei Verfahren (d) genannten Verdünnungsmittel. Verwendet man die Verbindungen der Formel (VII) in flüssiger Form, so ist es auch möglich, diese in entsprechendem Überschuß als Verdünnungsmittel einzusetzen.

Als Reaktionshilfsmittel zur Durchführung des erfindungsgemäßen Verfahrens (h) kommen tertiäre Amine, wie beispielsweise Triethylamin, N,N-Dimethylanilin, Pyridin, 4-(N,N-Dimethylamino)-pyridin, Diazabicyclooctan (DABCO), Diazabicyclonen (DBN) oder Diazabicycloundecen (DBU), in Frage.

Die Reaktionstemperaturen können bei der Durchführung des Verfahrens (h) in einem größeren Bereich variiert werden. Im allgemeinen arbeitet man zwischen -20 °C und +150 °C, vorzugsweise zwischen 0 °C und +100 °C.

Zur Durchführung des Herstellungsverfahrens (h) setzt man pro Mol 5-Amino-1-phenyl-pyrazol der Formel (Is) im allgemeinen 1,0 bis 20,0 Mol, vorzugsweise 1,0 bis 15,0 Mol an Verbindung der Formel (VII)-und gegebenenfalls 1,0 bis 3,0 Mol, vorzugsweise 1,0 bis 2,0 Mol an Reaktionshilfsmittel ein. Die Reaktionsdurchführung, Aufarbeitung und Isolierung der Reaktionsprodukte der Formel (Ih) erfolgt in allgemein üblicher Art und Weise.

Als Verdünnungsmittel zur Durchführung des erfindungsgemäßen Verfahrens (i) kommen inerte organische Lösungsmittel in Frage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (i) kann gegebenenfalls in Gegenwart eines geeigneten Säurebindemittels durchgeführt werden.

Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclonen (DBN) oder Diazabicycloundecen (DBU).

Es ist jedoch auch möglich, einen entsprechenden Überschuß an dem als Reaktionspartner eingesetzten Amin der Formel (IX) gleichzeitig als Säurebindemittel zu verwenden.

Die Reaktionstemperaturen können bei der Durchführung des erfindungsgemäßen Verfahrens (i) in einem größeren Bereich variiert werden. Im allgemeinen arbeitet man bei Temperaturen zwischen -20 °C und +200 °C, vorzugsweise bei Temperaturen zwischen 0 °C und +150 °C.

Zur Durchführung des erfindungsgemäßen Verfahrens (i) setzt man pro Mol an 5-Halogen-pyrazol der Formel (VIII) im allgemeinen 1,0 bis 10,0 Mol, vorzugsweise 1,0 bis 5,0 Mol an Amin der Formel (IX) ein. Die Reaktionsdurchführung, Aufarbeitung und Isolierung der Reaktionsprodukte der Formel (Ii) erfolgt nach allgemein üblichen Verfahren.

Als Verdünnungsmittel zur Durchführung der erfindungsgemäßen Verfahren (k) kommen inerte organische Lösungsmittel in Frage. Vorzugsweise verwendet man aliphatische, oder aromatische, gegebenenfalls halogenierte Kohlenwasserstoffe, wie Benzin, Benzol, Toluol, Xylol, Pentan, Hexan, Heptan, Cyclohexan, Petrolether, Ligroin, Methylenchlorid, Ethylenchlorid, Chloroform, Tetrachlorkohlenstoff,Chlorbenzol oder Dichlorbenzol, Ether, wie Diethylether oder Diisopropylether, Ethylenglykoldimethylether, Tetrahydrofuran oder Dioxan, Ketone, wie Aceton oder Butanon, Methylisopropylketon oder Methylisobutyl keton, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid, oder Alkohole wie Methanol, Ethanol oder Isopropanol.

Es ist jedoch auch möglich, die als Reaktionskomponenten verwendeten Verbindungen der Formel (X) in entsprechendem Überschuß gleichzeitig als Verdünnungsmittel einzusetzen.

Das erfindungsgemäße Verfahren (k) kann gegebenenfalls in Gegenwart eines basischen Katalysators durchgeführt werden. Als solche kommen alle üblichen anorganischen oder organischen Basen infrage. Vorzugsweise verwendet man die bei Verfahren (i) genannten Basen.

Die Reaktionstemperaturen können bei dem erfindungsgemäßen Verfahren (k) ebenfalls in einem größeren Bereich variiert werden. Im allgemeinen arbeitet man zwischen 0 °C und +200 °C, vorzugsweise zwischen +20 °C und +150 °C.

Zur Durchführung des erfindungsgemäßen Verfahrens (k) setzt man pro Mol (Bis)Carbamat der Formel (Iw) im allgemeinen 1 bis 20 Mol, vorzugsweise 1 bis 10 Mol der Verbindung der Formel (X) und gegebenenfalls 0,1 bis 2 Mol, vorzugsweise 0,1 bis 1 Mol Katalysastor ein und erwärmt für mehrere Stunden auf die erforderliche Temperatur. Die Aufarbeitung und Isolierung der Reaktionsprodukte der Formel (Ik) erfolgt nach üblichen Verfahren.

Als Verdünnungsmittel zur Durchführung der erfindungsgemäßen Verfahren (1) und (m) kommen polare organische Lösungsmittel, Wasser oder wäßrige Gemische, in Frage. Vorzugsweise verwendet man Alkohole, wie beispielsweise Methanol, Ethanol oder Propanol, deren Gemische mit Wasser oder reines Wasser.

Die Reaktionstemperaturen können bei der Durchführung der Herstellungsverfahren (1).und (m) ebenfalls in einem größeren Bereich variiert werden. Im allgemeinen arbeitet man zwischen 0 °C und +80 °C, vorzugsweise zwischen +20 °C und +40 °C.

Zur Durchführung der erfindungsgemäßen Verfahren (1) und (m) setzt man pro Mol 5-Amido-pyrazol der Formel (Ix) oder (Iy) im allgemeinen 1,0 bis 10 Mol, vorzugsweise 1,0 bis 5,0 Mol an Salz der Formel -(XI) oder an Amin ein.

Zur Herstellung der Natrium-, Kalium-oder Ammoniumsalze setzt man eine Verbindung der Formel (Ix) oder (Iy) in wäßriger Lösung oder einem organischen Lösungsmittel, wie Aceton, Methanol, Ethanol oder Dimethylformamid, mit Natrium-, Kalium-oder Ammoniumhyroxid oder einem Amin um und isoliert die Salze durch Abfiltrieren oder durch Eindampfen der Lösung und reinigt sie gegebenenfalls durch Umkristallisieren.

Die Calcium-, Barium-, Magnesium-, Mangan-, Kupfer-, Nickel-, Zinn-, Eisen-oder Cobaltsalze werden hergestellt aus den Natriumsalzen durch Behandeln mit einem entsprechenden anorganischen Metallsalz, z.B. Calciumchlorid, Bariumchlorid, Kupfersulfat, Nickelchlorid oder Cobaltnitrat. Die Calciumsalze können auch hergestellt werden durch Behandeln einer Verbindung der Formel (Ix) oder (Iy) mit Calciumhydroxid.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotina, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung dikotyler Unkräuter in monokotylen und dikotylen Kulturen wie beispielsweise Weizen oder Sojabohnen einsetzen.

Auch die Vorprodukte der Formel (VIII) besitzen eine hohe herbizide Wirksamkeit.

Außerdem zeigen die erfindungsgemäßen Wirkstoffe eine blattinsektizide Wirkung.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Sprizpulver, Suspensionen, Pulver, Stäubemittel, Pasten, lösliche Pulver, Granulate, Suspensions-Emulsions-Konzentrate, wirkstoffimprägnierte Natur-und synthetische Stoffe sowie Feinstverkapselungen in polaren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorben zole, Chlorethylene oder Methylenchlorid, aliphatische Kohlenwasserstoffe, wie Cyclohexan oder Paraffine,z.B. Erdölfraktionen, mineralische und pflanzliche Öle, Alkohole wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablauge und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2,-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischungen mit N,N-Dimethyl-N'-(3-chlor-4-methyl-phenyl)-harnstoff; N,N-Dimethyl-N'-(4-isopropylphenyl)-harnstoff; 2,4-Dichlorphenoxyessigsäure; 2,4-Dichlor phenoxypropionsäure; (2-Methyl-4-Chlorphenoxy)-essigsäure; (4-Chlor-2-methyl-phenoxy)-propionsäure; 2-[4-(3,5-Dichlorpyrid-2-yloxy)-phenoxy]-

propionsäure-(2-benzyloxyethylester); -(trimethylsilylmethylester) oder -(2,2-diethoxyethylester); Methyl-5-(2,4-dichlorphenoxy)-2-nitrobenzoat; 3,5-Diiod-4-hydroxybenzonitril; 2-Chlor-N-{[(4-methoxy-6-methyl-1,3,5-triazin-2-yl)-amino]-carbonyl}-benzolsulfonamid, 4-Ethylamino-2-t-butylamino-6-methylthio-s-triazin; N-Methyl-2-(benzthiazol-2-yloxy)-acetamid; N-(1-Ethylpropyl)-3,4-dimethyl-2,6-dinitroanilin; Chloressigsäure-N-(methoxymethyl)-2,6-diethylanilid; 2-Ethyl-6-methyl-N-(1-methyl-2-methoxyethyl)-chloracetanilid; 2,6-Dinitro-4-trifluormethyl-N,N-dipropylanilin; 2-{4-[[3-Chlor-5-(trifluormethyl)-2-pyridinyl]-oxy]-phenoxy}-propansäureethylester sowie weitere Triazinonen sind möglich. Einige Mischungen zeigen überraschenderweise auch synergistische Wirkung.

Auch Mischungen mit anderen bekannten Wirkstoffen, wie Fungiziden, Insektiziden, Akariziden, Nematiziden, Schutzstofen gegen Vogelfraß, Pflanzennährstoffen und Bodenstrukturverbesserungsmitteln sind möglich.

Die Wirkstoffe können als solche, in Form ihrer Formulierungen oder den daraus durch weeiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann bei der Anwendung der erfindungsgemäßen Verbindungen als Herbizide in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Bei der Anwendung der erfindungsgemäßen Verbindungen als Pflanzenwachtstumsregulatoren können die Aufwandmengen ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Herstellungsbeispiele:

Beispiel 1:

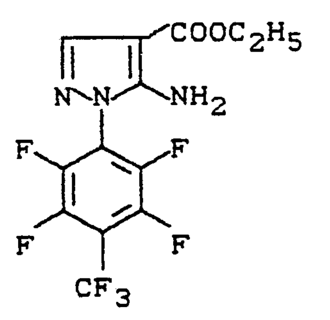

(Verfahren a)

40 g (0.161 Mol) 2,3,5,6-Tetrafluor-4-trifluormethylphenylhydrazin und 27,8 g (0.161 Mol) Ethoxymethylencyanessigsäureethylester werden in 70 ml Ethanol gelöst und 10 Stunden unter Rückfluß erwärmt. Das Lösungsmittel wird im Vakuum abdestestilliert und der Rückstand in 100 ml Diethylether suspendiert, filtriert und getrocknet. Man erhält 52,2 g (87,4 % der Theorie) 5-Amino-4-ethoxycarbonyl-1-(2,3,5,6-tetrafluor-4-trifluormethyl-phenyl)-pyrazol vom Schmelzpunkt 175 °C -176 °C.

Beispiel 2:

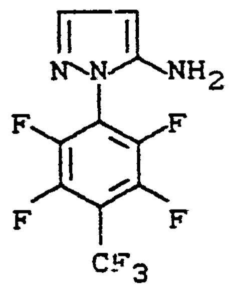

(Verfahren b + c)

100 g (0.270 Mol) 5-Amino-4-ethoxycarbonyl-1-(2,3,5,6-tetrafluor-4-trifluormethyl-phenyl)-pyrazol werden in 250 ml 50-prozentiger wässriger Schwefelsäure suspendiert und innerhalb von 2 Stunden auf 120 °C erwärmt; dabei werden die leichter flüchtigen Anteile über eine Brücke abdestilliert. Man hält weitere 3 Stunden auf 115 °C -120 °C, kühlt ab, verdünnt mit 400 ml Wasser, stellt mit verdünnter wässriger Natronlauge einen pH-Wert von 2 ein und saugt den entstandenen Niederschlag ab. Er wird mit Wasser neutral gewaschen und im Vakuum getrocknet. Man erhält 77,3 g (96 % der Theorie) 5-Amino-1-(2,3,5,6-tetrafluor-4-trifluormethyl-phenyl)-pyrazol vom Schmelzpunkt 103 °C -104 °C.

Beispiel 3:

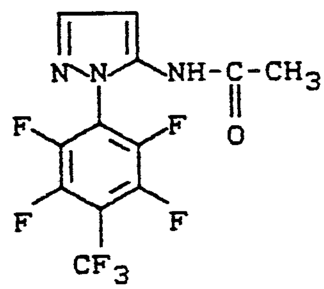

(Verfahren d)

In 60 g (0.20 Mol) 5-Amino-1-(2,3,5,6-tetrafluor-4-trifluormethyl-phenyl)-pyrazol in 170 ml Eisessig gibt man 250 ml (0.26 Mol) Essigsäureanhydrid, wobei die Reaktionstemperatur von 20 °C auf ca. 35 °C ansteigt. Man rührt 16 Stunden bei Raumtemperatur und trägt danach auf 800 ml Wasser aus. Der ausgefallene kristalline Feststoff wird abgesaugt, mit Wasser und Essigsäure frei gewaschen und im Vakuum getrocknet. Man erhält 59,8 g (87,7 % der Theorie) 5-Acetamido-1-(2,3,5,6-tetrafluor-4-trifluormethyl-phenyl)-pyrazol vom Schmelzpunkt 102 °C -104 °C.

Beispiel 4:

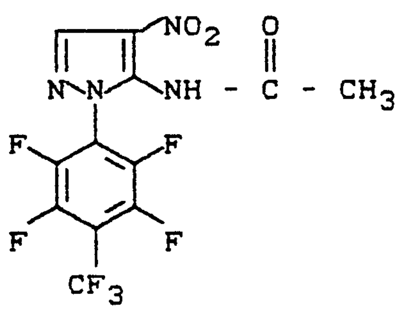

(Verfahren e)

Zu 34,1 g (0.10 Mol) 5-Acetamido-1-(2,3,5,6-tetrafluor-4-trifluormethyl-phenyl)-pyrazol in 85 ml Eisessig gibt man bei ca. 15 °C nacheinander 12,5 ml (0.13 Mol) Essigsäureanhydrid und 4,5 ml (0.105 Mol) 98-prozentige Salpetersäure. Man läßt die Temperatur langsam auf 25 °C steigen und rührt 4 Stunden nach. Anschließend wird die Reaktionslösung auf 250 ml Wasser ausgetragen. Der Niederschlag wird abfiltriert, neutral gewaschen und im Vakuum getrocknet. Man erhält 36,3 g (94 % der Theorie) 5-Acetamido-4-nitro-1-(2,3,5,6-tetrafluor-4-trifluormethyl-phenyl)-pyrazol vom Schmelzpunkt 138 °C -140 °C.

<u>Beispiel 5:</u>

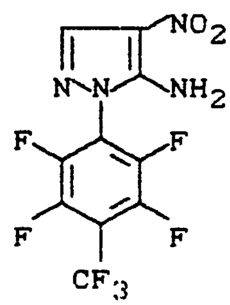

(Verfahren f)

19,3 g (0.05 Mol) 5-Acetamido-4-nitro-1-(2,3,5,6-tetrafluor-4-trifluormethyl-phenyl)-pyrazol werden in einer Mischung aus 40 ml 37-prozentiger Salzsäure und 70 ml Ethanol 7 Stunden unter Rückfluß erhitzt. Die Reaktionsmischung wird im Vakuum vom Ethanol befreit. Den Rückstand versetzt man mit 35 ml Wasser und neutralisiert mit verdünnter Natronlauge. Der Niederschlag wird abgesaugt, mit Wasser gewaschen und im Vakuum getrocknet. Man erhält 15,7 g (91,4 % der Theorie) 5-Amino-4-nitro-1-(2,3,5,6-tetrafluor-4-trifluormethyl-phenyl)-pyrazol vom Schmelzpunkt 123 °C -126 °C.

<u>Beispiel 6:</u>

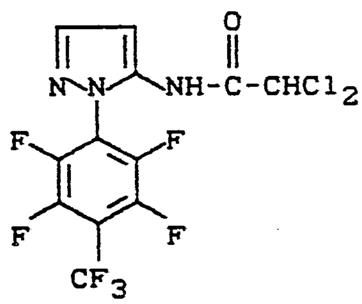

(Verfahren d)

Zu einer Lösung von 6 g (0.02 Mol) 5-Amino-1-(2,3,5,6-tetrafluor-4-trifluormethyl-phenyl)-pyrazol in 35 ml wasserfreiem Acetonitril gibt man nacheinander 1,8 ml (0.022 Mol) wasserfreies Pyridin und 2,1 ml - (0.021 Mol) 98-prozentiges Dichloracetylchlorid. Die Temperatur steigt auf 35 °C an. Man rührt vier Stunden nach und trägt danach auf 150 ml Wasser aus. Der ölige Niederschlag wird in 50 ml Dichlormethan aufgenommen, von der wässrigen Phase abgetrennt und nacheinander mit verdünnter Salzsäure, gesättigter Natriumhydrogencarbonat-und gesättigter Natriumchlorid-Lösung gewaschen, über Magnesiumsulfat getrocknet und im Vakuum vom Lösungsmittel befreit. Man erhält 7,0 g (85 % der Theorie) 5-Dichloracetamido-1-(2,3,5,6-tetrafluor-4-trifluormethyl-phenyl)-pyrazol vom Schmelzpunkt 112 °C -114 °C.

Beispiel 7:

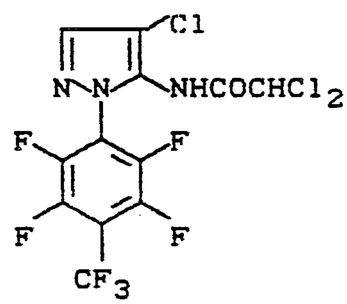

(Verfahren e)

Zu einer Lösung von 4,1 g (0.01 Mol) 5-Dichloracetamido-1-(2,3,5,6-tetrafluor-4-trifluormethyl-phenyl)-pyrazol in 20 ml Dichlormethan werden bei 0 °C -5 °C 0,9 ml (0.011 Mol) Sulfurylchlorid in 5 ml Dichlormethan zugetropft. Man rührt 16 Stunden bei Raumtemperatur, verdünnt mit 30 ml Dichlormethan, wäscht die Lösung nacheinander mit gesättigter Natriumhydrogencarbonat-und Natriumchlorid-Lösung, trocknet über Magnesiumsulfat und entfernt das Lösungsmittel im Vakuum. Man erhält 3,9 g (87,2 % der Theorie) 4-Chlor-5-dichloracetamido-1-(2,3,5,6-tetrafluor-4-trifluormethyl-phenyl)-pyrazol vom Schmelzpunkt 115 °C -116 °C.

Beispiel 8:

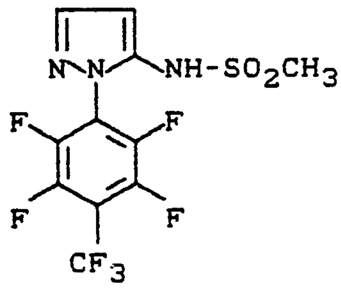

(Verfahren g)

Zu einer Lösung aus 6 g (0.02 Mol) 5-Amino-1-(2,3,5,6-tetrafluor-4-trifluormethyl-phenyl)-pyrazol in 30 ml wasserfreiem Pyridin gibt man bei 0 °C 3,2 ml (0.041 Mol) 99-prozentiges Methansulfonsäurechlorid. Es wird 16 Stunden bei Raumtemperatur gerührt und danach auf ca. 400 ml Eiswasser ausgetragen. Man versetzt mit 200 ml Dichlormethan, trennt die organische Phase ab, wäscht mit verdünnter Salzsäure und entfernt das Lösungsmittel im Vakuum. Der Rückstand wird in 75 ml Ethanol gelöst, mit 15 ml konzentrierter Ammoniaklösung versetzt und 24 Stunden bei 0 °C -5 °C gerührt. Das Ethanol wird im Vakuum entfernt, der Rückstand mit Dichlormethan extrahiert, die organische Phase nacheinander mit verdünnter Salzsäure, gesättigter Natriumhydrogencarbonat-und gesättigter Kochsalzlösung gewaschen. Nach der Entfernung des Lösungsmittels im Vakuum erhält man 4,1 g (54,2 % der Theorie) 5-Methansulfonamido-1-(2,3,5,6-tetrafluor-4-trifluormethylphenyl)-pyrazol als braunes Öl.

$^1$H-NMR($CDCl_3$) : $\delta$ = 3,04 s (3H), $\delta$=6,48 d (1H) und $\delta$ = 7,82 d (1H).

Beispiel 9:

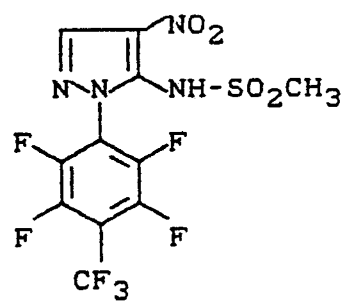

(Verfahren e)

Zu einer Lösung von 3,8 g (0.01 Mol) 5-Methansulfonamido-1-(2,3,5,6-tetrafluor-4-trifluormethyl-phenyl)-pyrazol in 20 ml Eisessig gibt man bei ca. 15 °C nacheinander 1,07 ml (0.011 Mol) Essigsäureanhydrid und 0,45 ml (0.0106 Mol) 98-prozentige Salpetersäure. Man rührt ca. 16 Stunden bei Raumtemperatur und trägt die Reaktionslösung auf 100 ml Wasser aus. Der Niederschlag wird abgesaugt, neutral gewaschen und im Vakuum getrocknet. Man erhält 3,4 g (80 % der Theorie) 5-Methansulfonamido-4-nitro-1-(2,3,5,6-tetrafluor-4-trifluormethyl-phenyl)-pyrazol vom Schmelzpunkt 163 °C -168 °C.

Beispiel 10:

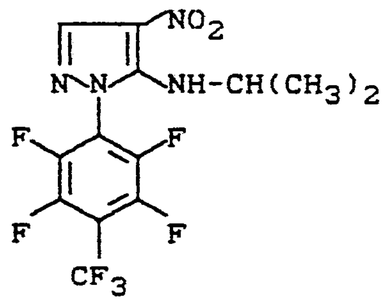

(Verfahren i)

Ein Gemisch aus 4,1 g (0.01 Mol) 1-(2,3,5,6-tetrafluor-4-trifluormethyl)-4-nitro-5-brom-pyrazol und 7,8 g (0.13 Mol) Isopropylamin in 100 ml Methylenchlorid wird 50 Stunden bei Raumtemperatur gerührt. Zur Aufarbeitung wird der Reaktionsansatz im Vakuum eingeengt, der Rückstand in 40 ml Methylenchlorid aufgenommen und mit 40 ml Wasser gewaschen. Die organische Phase wird über Natriumsulfat getrocknet und das Lösungsmittel im Vakuum abdestilliert. Der Rückstand wird durch Anreiben mit Petrolether zur Kristallisation gebracht, abgesaugt und getrocknet.

Das 1-(2,3,5,6-tetrafluor-4-trifluormethyl)-4-nitro-5-isopropylamino-pyrazol (Schmelzpunkt 98-100°C) wird durch präparative HPLC (High Pressure Liquid Chromatographie) aus dem Rohprodukt abgetrennt.

Herstellung des Ausgangsproduktes

Beispiel VIII-1:

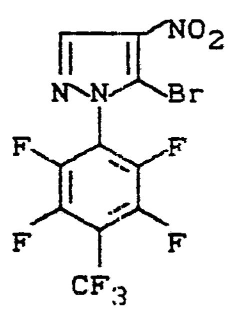

20,6 g (0.06 Mol) 5-Amino-4-nitro-1-(2,3,5,6-tetrafluor-4-trifluormethyl-phenyl)-pyrazol in 100 ml (1.125 Mol) Bromoform werden tropfenweise unter Rühren innerhalb von 10 Minuten mit 21,6 ml (0.18 Mol) t-Butylnitril versetzt, wobei die Temperatur der Reaktionsmischung auf 50 °C ansteigt. Nach beendeter Zugabe rührt man weitere zwei Stunden bei Rückflußtemperatur, engt die Mischung im Vakuum ein, nimmt den Rückstand in Methylenchlorid auf, wäscht mehrmals mit gesättigter Natriumbicarbonatlösung, 2n-Salzsäure und Wasser, trocknet über Natriumsulfat und zieht das Lösungsmittel im Vakuum ab. Man erhält 22 g (89,9 % der Theorie) 5-Brom-4-nitro-1-(2,3,5,6-tetrafluor-4-trifluormethyl-phenyl)-pyrazol als braunes Öl.

$^{1}$H-NMR($CDC1_3$) : $\delta$ = 8,5 ppm)

MS (Massenspektrum): $M^+$ 407 und 409

Beispiel 11:

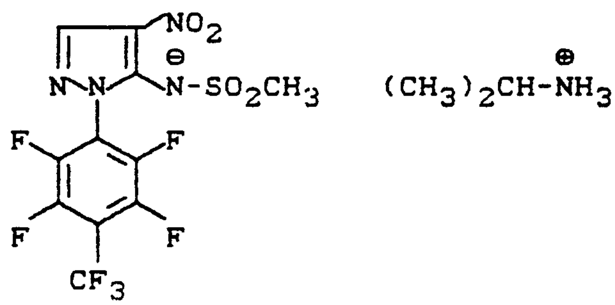

(Verfahren 1)

In einer Lösung von 5 g (0.012 Mol) 5-Methansulfonamido-4-nitro-1-(2,3,5,6-tetrafluor-4-trifluormethyl-phenyl)-pyrazol in 60 ml absolutem Ethanol gibt man 1,5 ml (0.0175 Mol) wasserfreiem Isopropylamin. Es wird eine Stunde bei Raumtemperatur gerührt, das Lösungsmittel im Vakuum entfernt, der glasige Rückstand in 50 ml Dichlormethan gelöst und die Lösung im Vakuum eingeengt. Man erhält 5 g (89 % der Theorie) des Isopropylammoniumsalzes vom Schmelzpunkt 60 °C -63 °C.

In entsprechender Weise und gemäß den allgemeinen Angaben zur Herstellung erhält man die folgenden 5-Amino-1-phenyl-pyrazole der allgemeinen Formel (I):

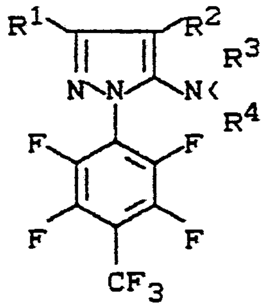

(I)

Tabelle 2

| Beisp. Nr. | $R^1$ | $R^2$ | $-N<^{R^3}_{R^4}$ | Schmelzpunkt/° C |
|---|---|---|---|---|
| 12 | H | H | $-NH-\overset{O}{\overset{\|}{C}}-C_2H_5$ | 115 - 117 |
| 13 | H | $NO_2$ | $-NH-\overset{O}{\overset{\|}{C}}-CHCl_2$ | 118 - 119 |
| 14 | H | H | $NH-COCF_3$ | 132 - 135 |
| 15 | H | H | $-NH-COCHCl-CH_3$ | 120 - 123 |
| 16 | H | H | $-NHCOC_3H_7$ | 56 - 59 |
| 17 | H | H | $-NHCOC_5H_{11}$ | 58 - 61 |
| 18 | H | H | $-NHCOCH_2-OCH_3$ | 109 - 113 |
| 19 | H | $NO_2$ | $-NHCOCH_2-OCH_3$ | 70 - 73 |
| 20 | H | H | $-NHCO-CHF_2$ | 158 - 160 |
| 21 | H | $NO_2$ | $-NHCOCF_3$ | 112 - 114 |
| 22 | H | $NO_2$ | $-NHCOC_3H_7$ | 98 - 99 |

Tabelle 2 (Fortsetzung)

| Beisp. Nr. | $R^1$ | $R^2$ | $-N\langle{R^3 \atop R^4}$ | Schmelzpunkt/°C |
|---|---|---|---|---|
| 23 | H | $NO_2$ | $-NH-COC_5H_{11}$ | 93 - 95 |
| 24 | H | $NO_2$ | $-NH-COCHClCH_3$ | 69 - 72 |
| 25 | 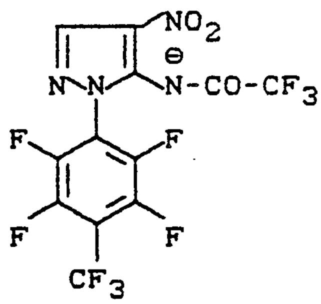 | | $(CH_3)_2CH-\overset{\oplus}{N}H_3$ | 123 - 128 |
| 26 | H | H | -NH-CO-H | 50-58 |
| 27 | H | H | $-NH-CO-CH_2Cl$ | 108-110 |
| 28 | H | $NO_2$ | -NH-CO-H | 160-163 |
| 29 | H | $NO_2$ | $-NH-CO-CH_2Cl$ | 83-84 |
| 30 | H | H | -NH-CO 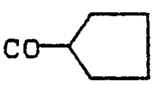 | 101-103 |
| 31 | H | $NO_2$ | $-NH-CO-OCH_3$ | Öl |
| 32 | H | $NO_2$ | -NH-CO 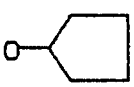 | 95-97 |

Anwendungsbeispiel:

In den folgenden Anwendungsbeispielen wurde die nachstehend aufgeführte Verbindung als Vergleichsubstanz eingesetzt:

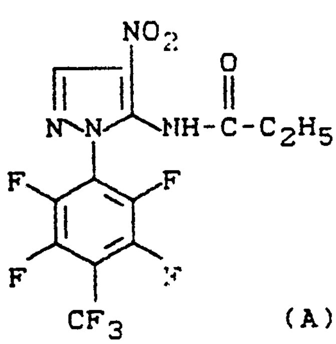

(A)

4-Nitro-5-propionamido-1-(2,3,5,6-tetrafluor-4-trifluormethyl-phenyl)-pyrazol (bekannt aus DE-OS 34 02 308)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber der Vergleichssubstanz (A) zeigen in diesem Test z.B. die Verbindungen gemäß den Herstellungsbeispielen: 1, 3, 5 und 24.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

Eine deutliche Überlegenheit in der Wirksamkeit sowie in der Nutzpflanzenselektivität gegenüber der Vergleichssubstanz (A) zeigen in diesem Test z.B. die Verbindungen gemäß den Herstellungsbeispielen: 1, 3, 5, 19 und 24.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
+ leichtes Austrocknen der Blätter, geringer Blattfall
+ + starkes Austrocknen der Blätter, starker Blattfall
+ + + sehr starkes Austrocknen der Blätter, sehr starker Blattfall

Eine deutliche Wirksamkeit im Vergleich zur unbehandelten Kontrolle zeigen in diesem Test beispielsweise die Verbindungen gemäß den Herstellungsbeispielen 2, 4, 7, 19, 21, 22, 23, 24, 28 und 29.

## Ansprüche

1. 5-Amino-1-phenyl-pyrazole der allgemeinen Formel (I),

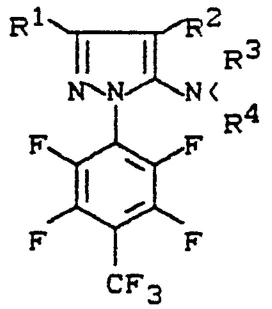

(I)

in welcher
$R^1$ für Wasserstoff oder für Alkyl mit 1 bis 12 Kohlenstoffatomen steht,
$R^2$ für Wasserstoff, Nitro, Nitroso, Halogen oder für einen Rest $-\underset{\underset{O}{\|}}{C}-R^5$
steht, wobei
$R^5$ für Wasserstoff, Hydroxy, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,
$R^3$ für Wasserstoff, für einen Rest
$-\overset{\overset{X}{\|}}{C}-R^6$ oder für einen Rest $-S(O)_n-R^7$, steht,
$R^4$ für Wasserstoff, für Alkyl, für einen Rest
$-\overset{\overset{X}{\|}}{C}-R^6$ oder für einen Rest $-S(O)_n-R^7$ steht, und für den Fall, daß $R^3$ für einen $-SO_2-R^7$-Rest oder einen $-CO-C_mF_{2m+1}$-Rest steht, auch für ein salzartig gebundenes anorganisches oder organisches Kation steht, wobei
$R^6$ für Wasserstoff, Alkyl, Alkenyl, Alkinyl, Halogenalkyl, Alkoxyalkyl, Alkylthioalkyl, Alkylsulfonylalkyl, Alkylsulfinylalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,
X für Sauerstoff oder Schwefel steht,
n für eine Zahl 0, 1 oder 2 steht,
m für eine Zahl 1, 2 oder 3 steht und
$R^7$ für Alkyl, Halogenalkyl oder für gegebenenfalls substituiertes Aryl steht,
wobei jedoch für den Fall, daß $R^1$ und $R^3$ für Wasserstoff stehen und $R^2$ für Nitro steht, $R^4$ nicht gleichzeitig für einen Propionylrest steht.

2. 5-Amino-1-phenyl-pyrazole der Formel (I) gemäß Anspruch 1, in welcher
$R^1$ für Wasserstoff oder für geradkettiges oder verzweigtes Alkyl mit 1 bis 8 Kohlenstoffatomen steht,
$R^2$ für Wasserstoff, Nitro, Nitroso, Fluor, Chlor, Brom, Iod oder für einen Rest $-\underset{\underset{O}{\|}}{C}-R^5$
steht,
wobei
$R^5$ für Wasserstoff, Hydroxy, geradkettiges oder verzweigtes Alkyl mit 1 bis 8 Kohlenstoffatomen, für jeweils geradkettiges oder verzweigtes Alkenyl, Alkinyl, Alkoxyalkyl, Alkylthioalkyl, Alkoxy, Alkylthio, Alkylamino,

Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten jeweils in Frage kommen: Halogen, jeweils geradkettiges oder verzweigtes Alkyl oder Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

$R^3$ für Wasserstoff, für einen Rest $-\underset{\underset{X}{\|}}{C}-R^6$

oder für einen Rest $-S(O)_n-R^7$ steht,

$R^4$ für Wasserstoff, für einen Rest $-\underset{\underset{X}{\|}}{C}-R^6$

oder für einen Rest $-S(O)_n-R^7$ steht, für geradkettiges oder verzweigtes Alkyl mit 1 bis 6 Kohlenstoffatomen steht, oder für den Fall, daß $R^3$ für einen Rest $-SO_2-R^7$ oder einen Rest $-CO-C_mF_{2m+1}$steht auch für ein salzartig gebundenes Äquivalent eines Alkali-oder Erdalkali-oder Übergangsmetallkations oder für ein gegebenenfalls einfach oder mehrfach, gleich oder verschieden durch $C_1$-$C_6$-Alkyl, Phenyl und Benzyl substituiertes Ammoniumion steht, wobei

$R^6$ für Wasserstoff, für geradkettiges oder verzweigtes Alkyl mit 1 bis 12 Kohlenstoffatomen, für jeweils geradkettiges oder verzweigtes Alkenyl oder Alkinyl mit jeweils 2 bis 4 Kohlenstoffatomen, für jeweils geradkettiges oder verzweigtes Alkoxyalkyl, Alkylthioalkyl, Alkoxy, Alkylthio, Alkylsulfonylalkyl, Alkylsulfinylalkyl, Alkylamino, Dialkylamino oder Halogenalkyl mit jeweils 1 bis 4 Kohlenstoffatomen in den einzelnen Alkylteilen und im Fall des Halogenalkyl mit bis zu 9 gleichen oder verschiedenen Halogenatomen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten jeweils in Frage kommen: Halogen, jeweils geradkettiges oder verzweigtes Alkyl oder Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

$R^7$ für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder für gegebenenfalls einfach oder mehrfach, gleich oder verschieden substituiertes Phenyl steht, wobei als Phenylsubstituenten in Frage kommen: Halogen, jeweils geradkettiges oder verzweigtes Alkyl, Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

X für Sauerstoff oder Schwefel steht,

n für eine Zahl 0, 1 oder 2 steht und

m für eine Zahl 1, 2 oder 3 steht,

wobei jedoch für den Fall, daß $R^1$ und $R^3$ für Wasserstoff stehen und $R^2$ für Nitro steht, $R^4$ nicht gleichzeitig für einen Propionylrest steht.

3. 5-Amino-1-phenyl-pyrazole der Formel (I) gemäß Anspruch 1, dadurch gekennzeichnet, daß

$R^1$ für Wasserstoff, Methyl, Ethyl, n-oder i-Propyl, n-, i-, s-oder t-Butyl steht,

$R^2$ für Wasserstoff, Nitro, Nitroso, Fluor, Chlor, Brom, Iod oder für einen Rest $-\underset{\underset{O}{\|}}{C}-R^5$ steht, wobei

$R^5$ für Wasserstoff, Hydroxy, Methyl, Ethyl, n-oder i-Propyl, n-, i-, s-oder t-Butyl, n-oder i-Pentyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 2-Chlorpropyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht,

$R^3$ für Wasserstoff, für einen Rest $-\underset{\underset{X}{\|}}{C}-R^6$

oder für einen Rest $-S(O)_n-R^7$ steht,

$R^4$ für Wasserstoff, für einen Rest

$-\overset{X}{\overset{\|}{C}}-R^6$ oder für einen Rest $-S(O)_n-R^7$ steht, für Methyl, Ethyl, n-oder i-Propyl, n-, i-, s-oder t-Butyl steht oder für den Fall, daß $R^3$ für Trifluormethylcarbonyl, Pentafluorethylcarbonyl, Heptafluor-n-propylcarbonyl, Heptafluor-i-propylcarbonyl oder für einen Rest $-SO_2-R^7$ steht, auch für ein salzartig gebundenes Äquivalent eines Natrium-, Kalium-, Magnesium-, Calcium-, Barium-, Kupfer-, Zink-, Mangan-, Zinn-, Eisen-, Cobalt- oder Nickelions steht, oder für ein gegebenenfalls ein-bis dreifach, gleich oder verschieden durch Methyl, Ethyl, n-oder i-Propyl, n-, i-, s-oder t-Butyl oder Phenyl substituiertes Ammoniumion steht, wobei

$R^6$ für Wasserstoff, Methyl, Ethyl, n-oder i-Propyl, n-, i-, s-oder t-Butyl, n-oder i-Pentyl, Hexyl, Heptyl, Octyl, Nonyl, Decyl, Undecyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 3-Chlorpropyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl, für jeweils gegegenenfalls ein-bis dreifach, gleich oder verschieden durch Methyl, Methoxy, Chlor oder Trifluormethyl substituiertes Phenyl, Phenoxy, Phenylthio oder Phenylamino steht,

$R^7$ für Methyl, Ethyl, n-oder i-Propyl, n-, i-, s-oder t-Butyl, Chlormethyl, Dichlormethyl, Trichlormethyl, Trifluormethyl oder für gegebenenfalls ein-bis dreifach, gleich oder verschieden durch Methyl, Methoxy, Chlor oder Trifluormethyl substituiertes Pehnyl steht,

X für Sauerstoff oder Schwefel steht,

n für eine Zahl 0, 1 oder 2 steht,

wobei jedoch für den Fall, daß $R^1$ und $R^3$ für Wasserstoff stehen und $R^2$ für Nitro steht, $R^4$ nicht gleichzeitig für einen Propionylrest steht.

4. 5-Amino-1-phenyl-pyrazole der Formel (I) gemäß Anspruch 1, in welcher

$R^1$ für Wasserstoff steht,

$R^2$ für Wasserstoff, Nitro, Nitroso, Fluor, Chlor, Brom, Iod oder für einen Rest $-\underset{O}{\underset{\|}{C}}-R^5$ steht,

wobei

$R^5$ für Wasserstoff, Methyl, Ethyl, n-oder i-Propyl, n-, i-, s-oder t-Butyl, n-oder i-Pentyl, für gegebenenfalls ein-bis dreifach, gleich oder verschieden durch Methyl, Methoxy, Chlor oder Trifluormethyl substituiertes Phenyl steht,

$R^3$ für Wasserstoff oder für einen Rest $-\underset{X}{\underset{\|}{C}}-R^6$ steht,

$R^4$ für Wasserstoff, für einen Rest $-\overset{X}{\overset{\|}{C}}-R^6$ oder für Methyl, Ethyl, n-oder i-Propyl, n-, i-, s-oder t-Butyl steht, wobei

$R^6$ für Wasserstoff, Methyl, Ethyl, n-oder i-Propyl, n-, s-, s-oder t-Butyl, n-oder i-Pentyl, Hexyl, Heptyl, Octyl, Nonyl, Decyl, Undecyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 3-Chlorpropyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht,

X für Sauerstoff oder Schwefel steht,

wobei jedoch die folgende Verbindung ausgeschlossen ist:

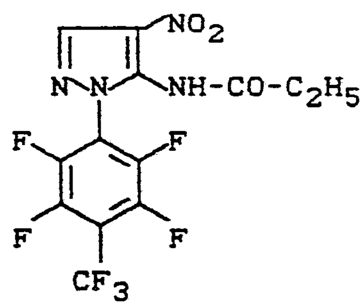

5. Das 5-Amino-1-phenyl-pyrazol-Derivat der Formel

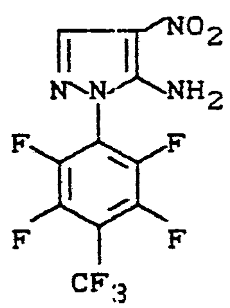

6. Verfahren zur Herstellung von 5-Amino-1-phenyl-pyrazolen der allgemeinen Formel (I),

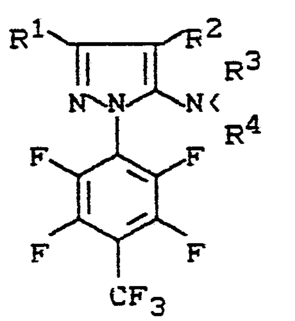
(I)

in welcher
$R^1$ für Wasserstoff oder für Alkyl mit 1 bis 12 Kohlenstoffatomen steht,
$R^2$ für Wasserstoff, Nitro, Nitroso, Halogen oder für den Rest $-\underset{\underset{O}{\|}}{C}-R^5$ steht, wobei
$R^5$ für Wasserstoff, Hydroxy, Alkyl, Alkenyl, Alkinyl, Halogenalkyl, Alkoxyalkyl Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,
$R^3$ für Wasserstoff, für einen Rest $-\overset{\overset{X}{\|}}{C}-R^6$ oder für einen Rest $-S(O)_n-R^7$ steht,
$R^4$ für Wasserstoff, für Alkyl, für einen Rest $-\overset{\overset{X}{\|}}{C}-R^6$ oder für einen Rest $-S(O)_n-R^7$ steht und für den Fall, daß $R^3$ für einen $-SO_2-R^7$-Rest oder einen $-CO-C_mF_{2m+1}$ Rest steht auch für ein salzartig gebundenes anorga-nisches oder organisches Kation steht,
$R^6$ für Wasserstoff, Alkyl, Alkenyl, Alkinyl, Halogenalkyl, Alkoxyalkyl, Alkylthioalkyl, Alkylsulfonylalkyl, Alkylsulfinylalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

X für Sauerstoff oder Schwefel steht,
n für eine Zahl 0, 1 oder 2 steht,
m für eine Zahl 1, 2 oder 3 steht und
$R^7$ für Alkyl, Halogenalkyl oder für gegebenenfalls substituiertes Aryl steht,
wobei jedoch für den Fall, daß $R^1$ und $R^3$ für Wasserstoff stehen und $R^2$ für Nitro steht, $R^4$ nicht gleichzeitig für einen Propionylrest steht,
dadurch gekennzeichnet, daß man:

a) die erfindungsgemäßen 5-Amino-1-phenyl-pyrazol-Derivate der Formel (Ia) erhält,

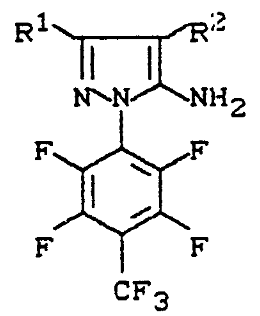

(Ia)

in welcher
$R^1$ und $R^2$ die oben angegebene Bedeutung haben,
wenn man 2,3,5,6-Tetrafluor-4-trifluormethylphenylhydrazin der Formel (II),

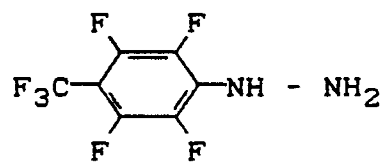

(II)

mit Acrylnitril-Derivaten der Formel (III),

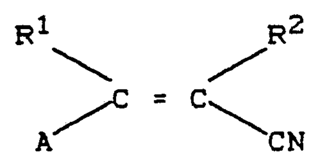

(III)

in welcher
$R^1$ und $R^2$ die oben angegebene Bedeutung haben und
A für Halogen, Hydroxy, Alkoxy oder Dialkylamino steht,
zunächst in einer 1. Stufe gegebenenfalls in Gegenwart eines Verdünnungsmittels und gegebenenfalls in Gegenwart eines Reaktionshilfsmittels umsetzt zu den Phenylhydrazin-Derivaten der Formel (IV),

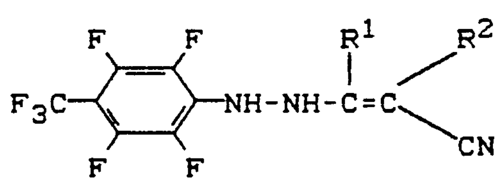

(IV)

in welcher
$R^1$ und $R^2$ die oben angegebene Bedeutung haben,
und diese in einer 2. Stufe, gegebenenfalls in Gegenwart eines Verdünnungsmittels und gegebenenfalls in Gegenwart eines Säurebindemittels cyclisiert,
oder daß man

b) die erfindungsgemäßen 5-Amino-1-phenyl-pyrazol-Derivate der Formel (Ib), erhält

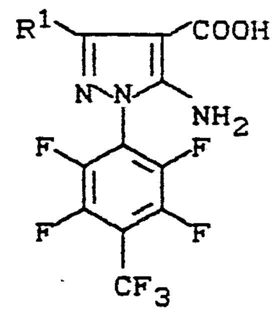

(Ib)

in welcher
$R^1$ die oben angegebene Bedeutung hat,
wenn man 4-Alkoxycarbonyl-5-amino-pyrazole der Formel (Ir)

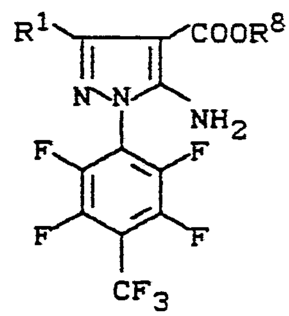

(Ir)

in welcher
$R^1$ die oben angegebene Bedeutung hat und
$R^8$ für Alkyl steht,
an der Estergruppe in 4-Position des Pyrazolringes in allgemein üblicher Art und Weise, gegebenenfalls in Gegenwart eines Verdünnungsmittels und gegebenenfalls in Gegenwart eines Katalysators, verseift,
oder daß man

c) die erfindungsgemäßen 5-Amino-1-phenyl-pyrazol-Derivate der Formel (Ic) erhält

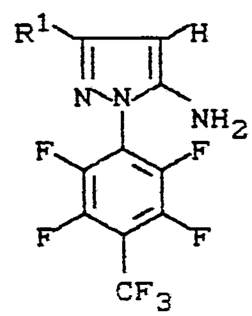

(Ic)

in welcher
$R^1$ die oben angegebene Bedeutung hat,
wenn man 5-Amino-1-phenyl-pyrazol-Derivate der Formel (Ib),

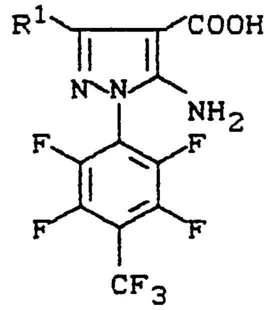

(Ib)

in welcher
$R^1$ die oben angegebene Bedeutung hat,
in allgemein üblicher Weise, gegebenenfalls in Gegenwart eines Verdünnungsmittels und gegebenenfalls in Gegenwart eines Katalysators decarboxyliert,
oder daß man

d) die erfindungsgemäßen 5-Amino-1-phenyl-pyrazol-Derivate der Formel (Id) erhält

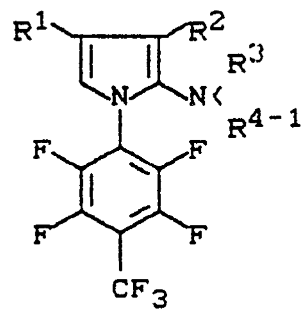

(Id)

in welcher
$R^{4-1}$ für Alkyl, für einen Rest
$-\overset{X}{\overset{\|}{C}}-R^6$ oder für einen Rest $-S(O)_n-R^7$ steht und
$R^1$, $R^2$, $R^3$, $R^6$, $R^7$, X und n die oben angegebene Bedeutung haben,
wenn man 5-Amino-1-phenyl-pyrazole der Formel (Is)

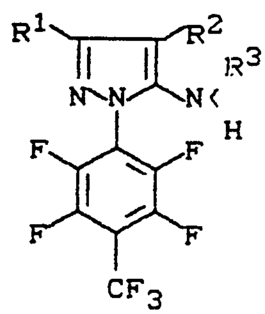

(Is)

in welcher $R^1$, $R^2$ und $R^3$ die oben angegebene Bedeutung haben,
(d-α) mit Verbindungen der Formel (V),

$R^6-\overset{X}{\overset{\|}{C}}-A^1$ (V)

in welcher
$A^1$ für Halogen oder für einen Rest
$R^6-\overset{O}{\overset{\|}{C}}-O-$steht und
$R^6$ und X die oben angegebene Bedeutung haben,

oder
(d-$\beta$) mit Verbindungen der Formel (Va),
$R^7$-$S(O)_n$-$A^2$ (Va)
in welcher
$A^2$ für Halogen steht und
$R^7$ und n die oben angegebene Bedeutung haben,
oder
(d-$\gamma$) mit Verbindungen der Formel (Vb),
$R^{8-1}$-$A^3$ (Vb)
in welcher
$R^{8-1}$ für Alkyl steht und
$A^3$ für Halogen, p-Toluolsulfonyloxy oder Alkoxysulfonyloxysteht,
gegebenenfalls in Gegenwart eines Verdünnungsmittels und gegebenenfalls in Gegenwart eines Säurebindemittels umsetzt,
oder daß man

e) die erfindungsgemäßen 5-Amino-1-phenyl-pyrazol-Derivate der Formel (Ie) erhält,

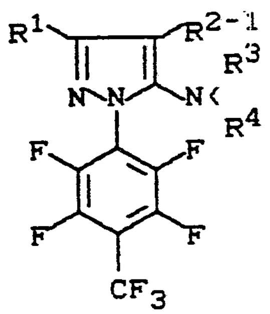

(Ie)

in welcher
$R^{2-1}$ für Halogen, Nitro, Nitroso, Formyl, Alkanoyl oder Aroyl steht und
$R^1$, $R^3$ und $R^4$ die oben angegebene Bedeutung haben,
wenn man 5-Amino-1-phenyl-pyrazol-Derivate der Formel (It),

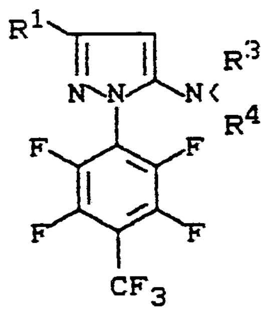

(It)

in welcher
$R^1$, $R^3$ und $R^4$ die oben angegebene Bedeutung haben,
mit elektrophilen Agenzien der Formel (VI),
$R^{2-1}$-$A^4$ (VI)
in welcher
$A^4$ für eine elektronenanziehende Abgangsgruppe steht und
$R^{2-1}$ die oben angegebene Bedeutung hat,
oder mit anderen üblichen elektrophilen Agenzien gegebenenfalls in Gegenwart eines Verdünnungsmittels und gegebenenfalls in Gegenwart eines Katalysators oder Reaktionshilfsmittels in 4-Stellung substituiert,
oder daß man

f) die erfindungsgemäßen 5-Amino-1-phenyl-pyrazol-Derivate der Formel (If) erhält

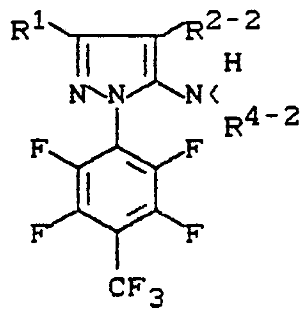

(If)

in welcher
$R^{2-2}$ für Halogen, Nitro oder Nitroso steht,
$R^{4-2}$ für Wasserstoff oder Alkyl steht und
$R^1$ die oben angegebene Bedeutung hat,
wenn man 5-Acylamino-1-phenyl-pyrazole der Formel (Iu),

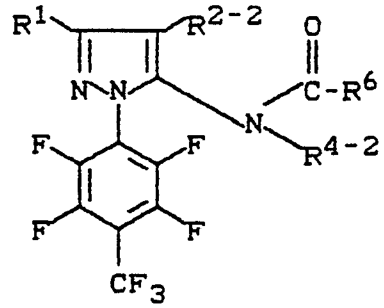

(Iu)

in welcher
$R^1$, $R^{2-2}$, $R^{4-2}$ und $R^6$ die oben angegebene Bedeutung haben,
gegebenenfalls in Gegenwart eines Verdünnungsmittels und gegebenenfalls in Gegenwart eines Katalysators an der Aminogruppe in 5-Position des Pyrazolringes deacyliert,
oder daß man

g) die erfindungsgemäßen 5-Amino-1-phenyl-pyrazol-Derivate der Formel (Ig) erhält

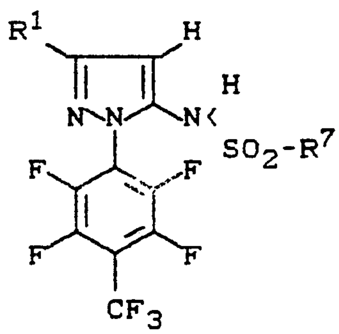

(Ig)

in welcher
$R^1$ und $R^7$ die oben angegebene Bedeutung haben,
wenn man 5-Bis-sulfonyl-amino-pyrazole der Formel (Iv),

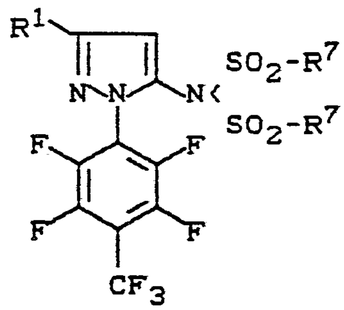

(Iv)

in welcher
$R^1$ und $R^7$ die oben angegebene Bedeutung haben,
mit Basen gegebenenfalls in Gegenwart eines Verdünnungsmittels spaltet,
oder daß man

h) die erfindungsgemäßen 5-Amino-1-phenyl-pyrazol-Derivate der Formel (Ih) erhält

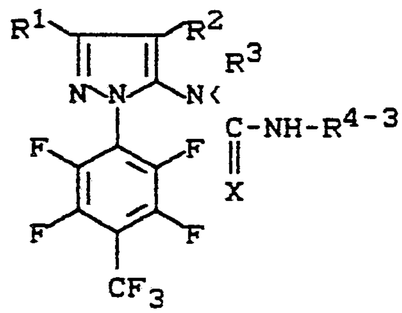

(Ih)

in welcher
$R^{4-3}$ für Alkyl oder für gegebenenfalls substituiertes Aryl steht und
$R^1$, $R^2$, $R^3$ und X die oben angegebene Bedeutung haben,
wenn man 5-Amino-1-phenyl-pyrazole der Formel (Is),

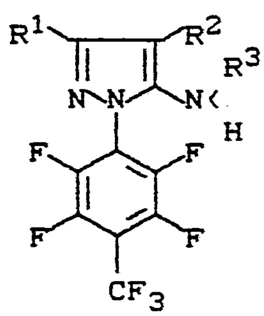

(Is)

in welcher
$R^1$, $R^2$ und $R^3$ die oben angegebene Bedeutung haben,
mit Iso(thio)cyanaten der Formel (VII),
$R^{4-3}$-N=C=X (VII)
in welcher
$R^{4-3}$ und X die oben angegebene Bedeutung haben,
gegebenenfalls in Gegenwart eines Verdünnungsmittels und gegebenenfalls in Gegenwart eines Säurebindemittels umsetzt,
oder daß man

i) die erfindungsgemäßen 5-Amino-1-phenyl-pyrazol-Derivate der Formel (Ii) erhält

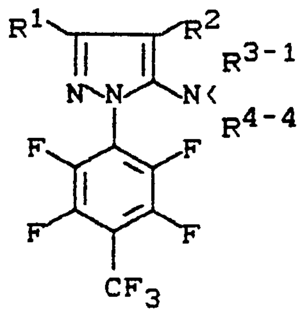

(Ii)

in welcher
$R^{4-4}$ für Alkyl steht,
$R^{3-1}$ für Wasserstoff oder Alkyl steht und
$R^1$ und $R^2$ die oben angegebene Bedeutung haben,
wenn man 5-Halogen-pyrazole der Formel (VIII),

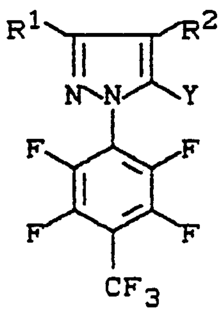

(VIII)

in welcher
Y für Halogen steht und
$R^1$ und $R^2$ die oben angegebene Bedeutung haben,
mit Aminen der Formel (IX),

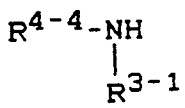

(IX)

in welcher
$R^{4-4}$ für Alkyl steht und
$R^{3-1}$ für Wasserstoff oder Alkyl steht,
gegebenenfalls in Gegenwart eines Verdünnungsmittels und gegebenenfalls in Gegenwart eines Säurebindemittels umsetzt,
oder daß man

k) die erfindungsgemäßen 5-Amino-1-phenyl-pyrazol-Derivate der Formel (Ik) erhält

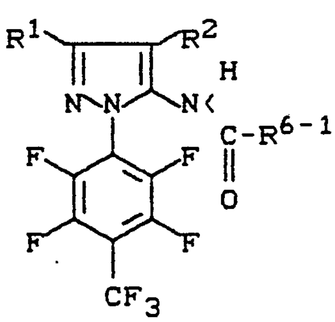

(Ik)

in welcher
$R^{6-1}$ für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht, und
$R^1$ und $R^2$ die oben angegebene Bedeutung haben,
wenn man (Bis)Carbamate der Formel (Iw),

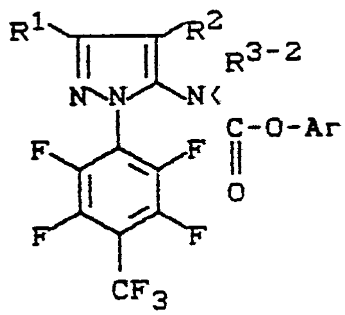

(Iw)

in welcher
$R^{3-2}$ für Wasserstoff oder für einen Rest
$-\overset{O}{\overset{\|}{C}}-O-Ar$ steht, wobei
Ar für gegebenenfalls substituiertes Aryl steht und
$R^1$ und $R^2$ die oben angegebene Bedeutung haben,
mit Verbindungen der Formel (X),
$R^{6-1}$ -H (X)
in welcher
$R^{6-1}$ die oben angegebene Bedeutung hat,
gegebenenfalls in Gegenwart eines Verdünnungsmittels und gegebenenfalls in Gegenwart eines basischen Katalysators umsetzt,
oder daß man

1) Salze von erfindungsgemäßen 5-Sulfonamido-pyrazol-Derivaten der Formel (Ix),
wenn man 5-Sulfonamido-pyrazole der Formel (Ix),

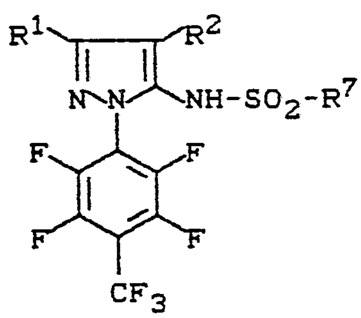

(Ix)

in welcher
$R^1$, $R^2$ und $R^7$ die oben angegebene Bedeutung haben,
entweder mit Salzen der Formel (XI),
$M^{\oplus}$ -$G^{\ominus}$ (XI)
in welcher
$M^{\oplus}$ für ein Äquivalent eines anorganischen oder organischen Kations steht und
$G^{\ominus}$ für ein Äquivalent eines geeigneten Gegenions steht,
oder mit primären, sekundären oder tertiären Aminen gegebenenfalls in Gegenwart eines Verdünnungsmittels umsetzt,
oder daß man

m) Salze von erfindungsgemäßen 5-Perfluoracylamido-pyrazol-Derivaten der Formel (Iy) erhält
wenn man 5-Perfluoracylamido-pyrazole der Formel (Iy),

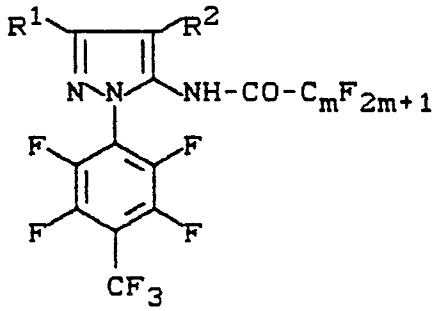

(Iy)

in welcher
$R^1$, $R^2$ und m die oben angegebene Bedeutung haben,
entweder mit Salzen der Formel (XI),
$M^{\oplus}$ -$G^{\ominus}$ (XI)
in welcher
$M^{\oplus}$ und $G^{\ominus}$ die oben angegebene Bedeutung haben,
oder mit primären, sekundären oder tertiären Aminen gegebenenfalls in Gegenwart eines Verdünnungsmittels umsetzt.

7. Herbizide und pflanzenwuchsregulierende Mittel, gekennzeichnet durch einen Gehalt an mindestens einem 5-Amino-1-phenyl-pyrazol der Formel (I) gemäß den Ansprüchen 1 bis 6.

8. Verfahren zur Bekämpfung von Unkräutern, dadurch gekennzeichnet, daß man 5-Amino-1-phenyl-pyrazole der Formel (I) gemäß den Ansprüchen 1 bis 6 auf die Unkräuter und/oder ihren Lebensraum einwirken läßt.

9. Verwendung von 5-Amino-1-phenyl-pyrazolen der Formel (I) gemäß den Ansprüchen 1 bis 6 zur Bekämpfung von Unkräutern und/oder als Pflanzenwachstumsregulatoren.

10. Verfahren zur Herstellung von herbiziden und/oder pflanzenwuchsregulierenden Mitteln, dadurch gekennzeichnet, daß man 5-Amino-1-phenyl-pyrazole der Formel (I) gemäß den Ansprüchen 1 bis 6 mit Streckmitteln und/oder oberflächenaktiven Substanzen vermischt.